(19) **Europäisches Patentamt**
**European Patent Office**
**Office européen des brevets**

(11) **EP 3 985 112 A1**

(12) **EUROPEAN PATENT APPLICATION**

(43) Date of publication:
**20.04.2022 Bulletin 2022/16**

(21) Application number: **21205118.9**

(22) Date of filing: **14.02.2020**

(51) International Patent Classification (IPC):
**C12N 15/10** $^{(2006.01)}$

(52) Cooperative Patent Classification (CPC):
**C12N 15/101**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.02.2019 GB 201902171**

(62) Document number(s) of the earlier application(s) in accordance with Art. 76 EPC:
**20707784.3 / 3 924 488**

(71) Applicant: **RevoluGen Limited**
**Hadfield**
**Derbyshire SK13 1QG (GB)**

(72) Inventor: **Patsos, Georgios**
**Hadfield, SK13 1QG (GB)**

(74) Representative: **Stafford, Jonathan Alan Lewis**
**Marks & Clerk LLP**
**1 New York Street**
**Manchester M1 4HD (GB)**

<u>Remarks:</u>
This application was filed on 27-10-2021 as a divisional application to the application mentioned under INID code 62.

(54) **PURIFICATION METHOD**

(57) Methods of isolating and purifying nucleic acid from a sample, and a filter for isolating nucleic acid from a sample, are described.

EP 3 985 112 A1

**Description**

**Field**

The present invention relates to a filter for isolating nucleic acid from a sample. The invention also relates to methods of isolating nucleic acid from a sample, a method of purifying nucleic acid isolated from a sample and a kit for isolating nucleic acid from a sample.

**Background**

The isolation of pure, intact nucleic acids comprising DNA from biological samples (e.g. cells) is of importance in a number of fields.

For example, clinical cell-containing samples from a patient may be subjected to a procedure (including the step of cell lysis) to isolate nucleic acids comprising DNA which is then analysed by procedures well known in the art. Such analysis may for instance be for the purpose of identifying DNA from a particular microbe, e.g. bacterium (to determine whether or not the patient has been infected by that microbe) or may for instance be for the purpose of whether or not the patient's DNA has one or more point mutations responsible for a medical condition.

Known methods of DNA isolation generally include at least the following steps:

(a) lysing the cell-containing sample with a buffered lysing composition;

(b) mixing the sample of (a) with a concentrated salt solution;

(c) mixing the sample from (b) with an alcohol (e.g. methanol, ethanol or iso-propanol);

(d) contacting the resultant mixture with a solid support (i.e. a filter) to immobilise DNA from the lysed material;

(e) washing the support with a wash liquid containing an alcohol; and

(f) eluting the DNA.

Such known methods of isolation often result in the extraction of relatively short DNA fragments, for example fragments of less than 50,000 base pairs (bp). These fragments can then be sequenced by either short-read sequencing technologies (e.g. those marketed by Illumina) or long-read sequencing technologies such as offered by Oxford Nanopore Technologies (ONT), for example Flow Cells ran on MinION, GridION X5, PromethION etc., or Pacific Biosciences (Pacbio) Single Molecule Real Time (SMRT) ran on the Sequel System. Short-read sequencing technologies are suitable for sequencing the code of relatively short fragments of DNA such 100 to 500bp. These sequences are then used to construct a genome based on overlapping sequence fragments. However, in many cases this type of technology cannot be used to decipher the code of repeat sequences, or recombined chromosome segments. The technology often also cannot be used to determine whether an aligned sequence originated from the same strand (chromosome) or cell, or whether the constructed genome is instead made of small reads from different strands and cells.

Long-read sequencing technologies can read sequences which are several tens or hundreds of thousands of bases long and so can be used to show that a sequence is derived from the same strand. In one clinically relevant example, long-read sequencing was used to successfully map mutations on the BCR-ABL1 fusion gene transcript. These types of mutations render Chronic Myeloid Leukaemia (CML) patients resistant to Tyrosine Kinase Inhibitor (TKI) therapy, and therefore it is important to characterise, screen and survey those mutations that are responsible for drug resistance. Due to the use of long-reads on a gene target of ~1,600bp (much larger than 100 to 500bp read by short read technologies), it has been possible to identify mutations that are found on the same strand and distinguish them from random alterations found on other strands or other transcript isoforms. This also provided information on CML mutation clonal distribution. These findings can educate the decision on which therapy to administer to the patient (Cavalier, et al. 2015).

Many long-read technologies require long DNA with an average of 100 kilobases (kb) or above. Even then, long-read technologies tend to bias towards reading smaller DNA fragments (under 10kb) if such fragments are present in a sample with longer fragments. This can lead to sequencing errors and difficulties in mapping the sequences. The bias towards short DNA fragments can also make it more difficult to target or read genes of complex genomes, such as the human genome.

However, it can be difficult to obtain good quality DNA which 1) contains long DNA fragments suitable for sequencing and 2) does not contain or has a reduced amount of shorter DNA fragments.

Factors affecting the ability of a given method to provide a given sample of particular nucleic acid include the ability of the solid support (i.e. filter) to retain the desired nucleic acids, and also the ability of the user to remove the relevant captured nucleic acids from the solid support / filter once captured, i.e. by elution from the filter / column.

Some methods of nucleic acid isolation use centrifugal force to isolate nucleic acids and/or remove impurities (spin method). Spin methods provide potentially quicker purification times due to the centrifugal force; however, such methods are typically not used for isolating long chain nucleic acids. This is because the centrifugal force required to extract nucleic acid of a suitable yield and purity for sequencing or other downstream assays has a tendency to fragment the nucleic acid, reducing chain length. Other methods for isolating nucleic acid isolation utilise anion exchange resins and the force of gravity to isolate nucleic acids and/or remove impurities. Although this is gentler than spin methods, processing can take significant amounts of time and so is not particularly user-friendly. This hinders the ability to use such methods in a point of care scenario. In addition, anion exchange resins isolate short and long nucleic acid fragments within the same mix.

Alternative methods for the extraction of DNA which avoid spinning steps include magnetic bead or magnetic flat surface technologies. With such technologies, great care must be taken by the user not to disturb the bead pellet when aspirating liquids. Thus, the extraction protocol is user-intensive and cannot be multiplexed. In addition, magnetic bead and magnetic flat surface technologies do not offer the capacity of size exclusion during the process of DNA extraction.

There therefore exists a need for new filters and processes for the extraction of nucleic acid from a sample which give higher yield, and/or higher purity of longer nucleic acid fragments and/or to provide samples with greater ratios of longer to shorter fragments. There also exists a need for the improved provision of longer nucleic acid fragments in a method which is faster and user-friendly.

The present invention seeks to address one or more of the above-mentioned issues.

**Summary of invention**

At its most general, the present disclosure proposes a new filter for isolating nucleic acids from a sample, the filter having particular utility in providing product samples containing a higher ratio of longer to shorter nucleic acid fragments and/or an increased average base pair length of nucleic acid fragments. The filter of the invention has broad applicability in methods of isolating nucleic acids from a sample and as such may be used in methods of spin purification or conventional gravity purification, but with particularly advantageous application in spin purification methods. Filters in this context are used in the art and according to methods/uses of the invention to capture/retain the desired nucleic acids from fluid samples brought in contact with the filters (usually passed through the filters) whilst the undesired components of the formulation (which may comprise undesired fragments of nucleic acids or other undesired components of the sample, e.g. other biological products) are allowed to pass through the filter in the filtrate. In this regard, the present invention is able to provide improved isolation of longer versus shorter fragments of nucleic acids. The present disclosure also proposes new processes for isolating nucleic acids. The processes have general applicability to the filters of the invention described above, as well as conventional filters used in the art. The present new processes are in particular able to provide one or more of an increased yield of recovery of purified nucleic acids (particularly those of longer chain lengths), an increased ratio of longer to shorter chain nucleic acids and an increased average base pair length of isolated nucleic acid.

The present disclosure thus provides the following aspects of the invention. These aspects, and general and specific embodiments of these aspects are described further below in the detailed description of the invention section.

According to a first aspect of the invention, there is provided a filter for isolating nucleic acid from a sample, the filter comprising at least a first porous region and a second porous region, the first porous region being arranged to be contacted in use by the sample before the second porous region, wherein the first porous region has a nominal pore size which is greater than the second porous region.

According to a second aspect of the invention there is provided a nucleic acid extraction column comprising the filter according to the first aspect.

According to a third aspect of the invention, there is provided a method of isolating nucleic acid from a sample using a filter according to the first aspect of the invention. Put another way, the present invention also provides the use of a filter according to the first aspect of the invention in a method of isolating nucleic acid from a sample. In embodiments, the method comprises:

(i) contacting a sample comprising nucleic acid with an alcohol and a salt (optionally by contacting the sample with a solution containing the alcohol and the salt) (i.e. to provide a composition containing the nucleic acid, alcohol and salt), the salt is optionally provided as a salt solution at a concentration of 0.05 to 10M; and

(ii) contacting a filter according to the first aspect of the invention with the resulting composition of step (i) to provide a filter having nucleic acid bonded thereto; and

(iii) preferably treating the filter having the nucleic acid bonded thereto with a wash solution comprising alcohol; and

(iv) eluting at least a portion of the nucleic acid from the filter.

According to a fourth aspect of the present invention, there is provided a method of isolating nucleic acid from a sample, the method comprising:

(i) contacting a sample comprising nucleic acid with an alcohol and a salt (optionally a solution comprising the alcohol and salt) (i.e. to provide a composition containing the nucleic acid, alcohol and salt), the salt is optionally provided as a salt solution at a concentration of 0.05 to 10M; and

(ii) contacting a filter with the composition comprising nucleic acid obtained following step (i) to provide a filter having nucleic acid bonded thereto; and

(iii) preferably, treating the filter having the nucleic acid bonded thereto with a wash solution comprising alcohol; and

(iv) eluting at least a portion of the nucleic acid from the filter, wherein elution comprises incubating the filter in an elution buffer at a temperature of from 70 to 90°C.

According to a fifth aspect of the present invention, there is provided a method of purifying nucleic acid isolated from a sample, the method comprising:

(i) contacting the nucleic acid with an alcohol and a salt (optionally wherein the alcohol and salt are provided in a solution) (i.e. to provide a composition containing the nucleic acid, alcohol and salt), the salt optionally being provided at a concentration of 0.05 to 10M;

(ii) contacting a filter with the composition comprising nucleic acid obtained following step (i) to provide a filter having nucleic acid bonded thereto;

(iii) preferably, treating the filter having the nucleic acid bonded thereto with a wash solution comprising alcohol (the alcohol solution optionally further containing a salt such as a lithium salt, optionally at a concentration of from 0.05M-10M); and

(iv) carrying out a first elution of nucleic acid from the filter, wherein the elution comprises incubating the filter in an elution buffer at a temperature of from 70 to 90°C followed by centrifugation of the filter; and

(v) carrying out a second elution of nucleic acid from the filter, wherein elution comprises incubating the filter in an elution buffer at a temperature not exceeding 90°C (e.g. from 10-90 °C) followed by centrifugation of the filter.

According to another aspect of the invention, there is provided a kit for isolating nucleic acid from a sample, the kit comprising:

(i) a filter according to the first aspect;
(ii) a salt (preferably a chaotropic agent, for example a guanidinium salt),
(iii) a wash agent, optionally comprising lithium salt;
(iv) optionally, an elution buffer; and
optionally a lysing agent.

**Detailed description of the invention**

According to a first aspect of the invention, there is provided a filter for isolating nucleic acid from a sample, the filter comprising at least a first porous region and a second porous region, the first porous region being arranged to be contacted in use by the sample before the second porous region, wherein the first porous region has a nominal pore size which is greater than the second porous region.

The present inventor has surprisingly found that this combination of porous regions in the filter leads to a greatly improved retention of longer nucleic acid fragments relative to shorter fragments on the filter. Embodiments of the invention are able to provide increased yields of total isolated nucleic acid, an increased average base pair length of isolated nucleic acid and/or provide an increased ratio of longer to shorter nucleic acid in the sample(s) that can be eluted from the filter following the filtration process.

The nucleic acid may be any form of nucleic acid (such as RNA and/or DNA) and is preferably DNA. In embodiments, the filter is able to increase the retention of nucleic acid fragments, of 10 kb or greater, such as 10 kb to 165.5 kilobases (kb) in length relative to nucleic acids of shorter lengths, e.g. relative to fragments of less than 10kb in length. In some embodiments, the filter of the invention is able to demonstrate increased retention of nucleic acid fragments of 40 kb or greater, such as from 40 to 165.5 kb in length relative to shorter fragments, e.g. relative to fragments of less than 40kb in length.

The filter is able to increase the retention of nucleic acid fragments having an increased average base pair length relative to filters not of the invention.

Although porous regions having a smaller nominal pore size are understood to be beneficial for the isolation of any length of nucleic acid fragment, the resulting isolated nucleic acid fragments are of a variety of lengths, including smaller and longer fragments. The smaller fragments in the isolated nucleic acid can interfere with downstream processing where larger fragments are desirable, such as sequencing.

Since porous regions comprising a smaller pore size are beneficial for retaining all sizes of fragments, the skilled person would expect the filter of the present invention, which comprises a porous region comprising a smaller nominal pore size, to equally retain and thus provide isolated nucleic acid fragments of a variety of lengths. It is therefore surprising that the filter of the present invention provides an increased average base pair length of isolated nucleic acid and/or provides an increased ratio of longer to shorter nucleic acid in the isolated sample(s)

Smaller nominal pore sizes are also considered to be less effective for the isolation of longer nucleic acid fragments, potentially due to blockage of the pores by other molecules in biological samples, such as proteins, lipids and/or carbohydrates which may not be fully denatured. This may result in tearing of the porous region comprising the smaller nominal pores, which can result in the loss of nucleic acid fragments retained on the porous region. This may additionally or alternatively result in coverage of the porous region by the other molecules which can remove surface area of the porous region for the longer nucleic acid fragments to bind to.

Thus, it is especially surprising that the combination of a first porous region having a greater pore nominal pore size and a second porous region having a smaller nominal pore size downstream of the first region provides at least one of an increased yield of total isolated nucleic acid, an increased average base pair length of isolated nucleic acid and an increased ratio of longer to shorter nucleic acid in the isolated sample(s).

While porous regions having a greater nominal pore size are traditionally thought to be more beneficial for the isolation of long nucleic acid fragments, the inventor has observed that porous regions having a greater pore size tend to display a weaker structural integrity and so are more prone to breakage, leakage, and loss of nucleic acid fragments retained compared to porous regions having a smaller pore size. The skilled person would therefore appreciate that for nucleic acid filters comprising porous regions for use in such purification methods, the benefits of providing larger pores with respect to nucleic acid isolation are negatively impacted by the reduced structural integrity, meaning that both the total yield of nucleic acid retained by the filter and the selectivity for longer fragments can be impacted.

The inventor has previously found that a filter of the present invention provides improved structural integrity relative to a comparative filter. The filter of the present invention is identical to the comparative filter except that the second region of the filter of the invention has smaller pores relative to the first region. This improved integrity makes the filter of the present invention easier to handle and to use in practice. In addition, the filter of the invention is less prone to tearing.

The inventor has surprisingly discovered that by providing a filter having first (upstream) region of larger pore size in combination with a second (downstream) region of smaller pore size that improved results are observed with respect to providing elution batches that contain longer chain fragments, e.g. by virtue of yield and / or ratio of long to short fragments in such elution batches.

Without wishing to be bound by theory, the present inventor believes that the improvement observed by filters of the invention is because the second porous region provides additional structural integrity to the first region. This ensures that the first porous region is better able to retain its functional ability to bind longer nucleic acid fragments compared to comparative filters which do not have the additional improvement in integrity.

Surprisingly, the present inventor has also found that the order of the porous regions affects the yield, ratio and average base pair length of nucleic acid fragments isolated; arranging the first porous region (having a larger nominal pore size) to be contacted by the sample in use prior to the second porous region (having a smaller nominal pore size) provides an improved yield of total isolated nucleic acid. The ratio of long to short nucleic acid obtained as well as the average base pair length is also improved. This is surprising as the tendency of the skilled person would be to think that the smaller-pore region may adversely affect the yield and / or selectivity of the filter for longer nucleic acids, but the inventor has found this not to be the case.

Without wishing to be bound by theory, the present inventor believes that shorter nucleic acid fragments bind faster to a porous region than longer nucleic acid fragments. By arranging a porous region with a greater pore size first (such that this region is contacted by the sample before the sample passes through the second region), the larger nucleic acid fragments may be preferentially captured over the short nucleic acid fragments, the shorter nucleic acid fragments instead being more likely to pass through the filter device without binding.

It will be appreciated that any of the features described herein (including any accompanying claims and drawings), may be combined with any of the below aspects in any combination, unless otherwise indicated.

As the skilled person will appreciate, nominal pore size is a known term of the art. The value of the nominal pore size indicates the ability of the porous region to filter out particles having that size value or greater. For example, a 0.20μm nominal pore size is used to indicate that the porous region having that pore size can prevent particles of 0.20μm or greater from passing through the porous region. Typically, the nominal pore size has a minimum percentage, i.e. the nominal pore size relates to the ability of the porous region to filter out a minimum percentage of particles having that size value or greater. In some embodiments, the nominal pore size has a minimum percentage of at least 50, at least 60, at least 70, at least 80 or at least 90%, i.e. the percentage of particles of a given size value or greater which the porous region having the particular nominal pore size filters out. In some embodiments the nominal pore size has a minimum percentage of at least 95%. In some embodiments the nominal pore size has a minimum percentage of at least 98% (i.e. the porous region is able to filter out at least 95% or at least 98% respectively of particles having the given particle size).

Various methods are suitable for determining the nominal pore size of a porous region. In some embodiments the method for determining the nominal pore size of a porous region comprises porosimetry. This method is based on liquid being held in the pores of a porous region by surface tension and capillary forces. The minimum pressure required to force liquid out of the pores against the opposing force of the liquid's surface tension can be used as a measure of the pore diameter. The method is generally performed within a vacuum, for example in a porosimeter, with an increasing force applied in the form of a compressed gas. Numerous porosimeters are available commercially and so are readily accessible to the skilled person.

Generally, a non-wetting liquid such as mercury is used as the liquid in porosimetry.

Washburn's equation is generally used to determine the force balance in porosimetry methods. This is:

$$P_L - P_G = - \frac{4\sigma \cos \theta}{D_P}$$

$P_L$ = pressure of liquid
$P_G$ = pressure of gas
$\sigma$ = surface tension of liquid
$\theta$ = contact angle of liquid
$D_p$ = pore diameter

The contact angle of mercury with most solids is approximately 140°, while the surface tension of mercury at 20°C under vacuum is 480 mN/m. Based on these values, the equation becomes:

$$D_P = \frac{1470 \text{ kPa . μm}}{P_L}$$

Other suitable tests for determining pore size include visual examination using scanning electron microscopy. Thus, in some embodiments, the method for determining the nominal pore size of a porous region comprises scanning electron microscopy. In such tests, at least a portion of the porous region is viewed under a scanning electron microscope (SEM) to obtain SEM images of the section. These images can then be quantitatively analysed, for example using imaging software such as ImageJ (NIH, US) to measure pore size. A mean or median pore size can then be calculated. From this, the skilled person can then determine what size of particle would be able to pass through this pore size, and thus the nominal pore size.

In some embodiments, particle challenge is used to determine the nominal pore size of a porous region. In this method, particles of a known size, often termed "challenge particles", are applied to a porous region. The particle size and abundance of particles are then measured up and downstream of the porous region. Measurement may be by microscopy, automated particle counters, turbidometry flow cytometry, submicron particle counters or other light scattering based techniques, depending on the size of the particle and whether or not it is labelled with a detection marker, for example a fluorescent marker.

In some embodiments the first region may be preceded by a further porous region which may have a different or a substantially equal nominal pore size, e.g. identical, to the nominal pore size of the first porous region. In embodiments where the further preceding porous region has a substantially equal nominal pore size to the nominal pore size of the first porous region, the preceding porous region may be understood to be a first porous region, e.g. part of the first porous region. In some embodiments, the first region is not preceded by a further porous region (i.e. the first porous region is the first porous region of the filter which contacts the nucleic acid composition in use).

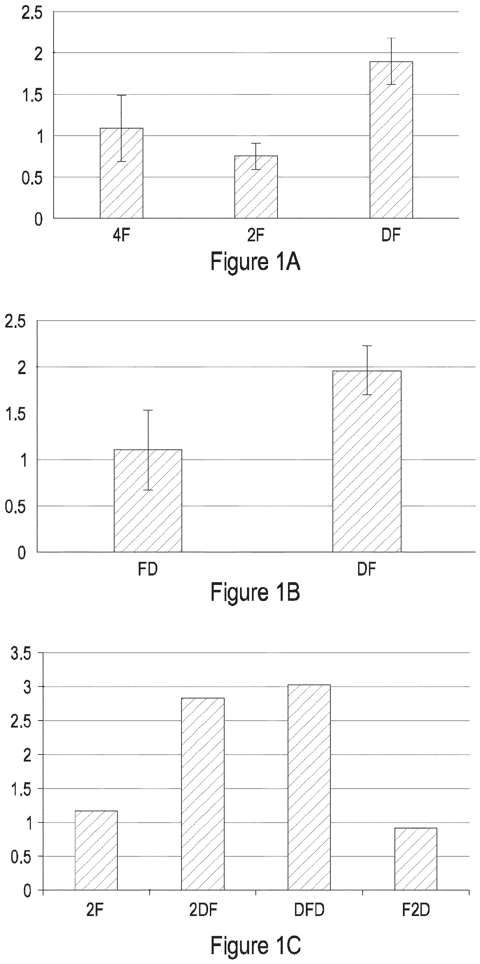

Thus, in some embodiments the filter device comprises a plurality of first porous regions. It will be appreciated that in such embodiments, the plurality of first porous regions will be adjacent to each other in the direction of fluid flow. This arrangement has been found to be especially beneficial for isolating an improved ratio of longer to shorter nucleic acid fragments (see, e.g. Figure 1C).

In other embodiments the further preceding porous region has a nominal pore size which is smaller than the nominal pore size of the first porous region. Optionally, the further preceding porous region has a nominal pore size which is substantially equal, e.g. identical, to the nominal pore size of the second porous region.

In some embodiments the filter comprises a further porous region (e.g. a third porous region), which is arranged to be contacted in use by the sample after the second porous region. It will be appreciated that the third porous region may have a substantially equal (e.g. identical) or a different nominal pore size to the second porous region.

The third porous region may have a substantially equal (e.g. identical) or a different nominal pore size to the first porous region.

In some embodiments the third porous region has a nominal pore size which is greater than the second porous region, optionally a nominal pore size which is substantially equal to the nominal pore size of the first porous region. The inventor has found the arrangement of the third and the first porous regions having a substantially equal nominal pore size to be particularly beneficial in the isolation of an increased ratio of longer to shorter nucleic acid fragments and an increased average base pair length of isolated nucleic acid fragments.

In some embodiments the nominal pore size of the first porous region is at least 2μm. The nominal pore size of the first porous region may in embodiments be no more than 3μm. Optionally, the nominal pore size of the first porous region is from 2μm to 3μm. This nominal pore size range has been found by the inventor to be advantageous in retaining longer nucleic acid fragments, for example nucleic acid fragments of 40 kb or greater, such as from 40 to 165.5 kb. In some embodiments the nominal pore size of the first region is from 2.5 to 2.8μm, optionally around 2.7μm, e.g. 2.7 μm.

The nominal pore size of the second porous region may be at least 0.5μm. In some embodiments the nominal pore size of the second porous region is no more than 1.8μm. Optionally, the nominal pore size of the second porous region is from 0.5 to 1.8μm. In some embodiments the nominal pore size of the second porous region is from 0.5μm to 1.3μm.

In some embodiments the nominal pore size of the second porous region is from 0.6 to 1 μm. The nominal pore size of the second porous region may be around 0.7 μm, e.g. may be 0.7 μm.

In some embodiments, the nominal pore size of the first porous region is at least 2 μm (and optionally no more than 3 μm) and the nominal pore size of the second porous region is no more than 1.8 μm, e.g. optionally from 0.5 to 1.8 μm. For instance, in some embodiments, the nominal pore size of the first region is from 2.5 to 2.8 μm, preferably around 2.7 μm, and the nominal pore size of the second porous region is from 0.5 to 1.8 μm, optionally from 0.6 to 1 μm, preferably around 0.7 μm.

Various embodiments of the invention comprise a nominal pore size of the second region of around 0.7μm (e.g. 0.7 μm) and a nominal pore size of the first region of around 2.7 μm (e.g. 2.7 μm).

The filters described herein may comprise any suitable material known to the skilled person to be useful for retaining nucleic acids. Suitable materials known for this purpose include silicates. Thus, in some embodiments at least one of the first and the second porous regions comprises (e.g. consists of) a silicate or silica-based material. In embodiments comprising a third and/or further porous region, the third and/or further porous region may comprise (e.g. consist of) a silicate or silica-based material. In suitable embodiments, all respective porous regions may comprise (e.g. consist of) silicate or silica-based material. Preferred silicate or silica-based materials include silicate glass, for example borosilicate or quartz glass, most preferably borosilicate glass.

The pores in filters of the invention may be provided by a number of methods known to the skilled person. Typically however, the pores are provided as apertures formed between particles of a packed fibrous material, e.g. in the form of a compacted fibrous mesh. In some embodiments, the filter comprises (e.g. is composed of, or consists of) a fibrous material, such as any of the materials mentioned in the preceding paragraph, for instance silicate glass fibres, for example borosilicate glass fibres or quartz glass fibres, preferably borosilicate glass. As such, the first porous region and / or the second porous region may be formed of the fibrous material, optionally comprising (or consisting of) fibres of a silica or silica-based material, such as silicate glass fibres, for example borosilicate glass fibres or quartz glass fibres. In some embodiments at least one of the first and the second porous region comprise borosilicate glass fibres. The first porous region and the second porous region may be formed of the same or a different fibrous material, e.g. borosilicate glass fibres or quartz glass fibres, for example QM-A quartz glass microfibres. In embodiments, each of the respective porous regions of the filter of the invention comprises (e.g. is composed of, or consists of) a fibrous material, such as any of the materials mentioned in the preceding paragraph, for instance silicate glass fibres, for example borosilicate glass fibres or quartz glass fibres, preferably borosilicate glass.

In embodiments comprising a third and/or a further porous region, the third and/or further porous region may be formed of a fibrous material, optionally comprising (or consisting of) fibres of a silica or silica-based material, such as silicate glass fibres, for example borosilicate glass fibres or quartz glass fibres. The third and/or further porous region may be formed of the same and or different fibrous material to the second and/or the first porous region.

In some embodiments, the filter comprises quartz glass fibres, for example QM-A quartz glass microfibre.

In some embodiments each of the first and second porous regions comprises a membrane or sheet of porous material.

Filters of the type available under the designation Whatman Glass Microfibre filters (GF) are suitable for use in the invention. Examples of such filters include GF filters of grade A, B, D and F. Grade A filters have a nominal pore size of 1.6 $\mu$m. Grade B filters have a nominal pore size of 1.0 $\mu$m. Grade D filters have a nominal pore size of 2.7 $\mu$m. Grade F filters have a nominal pore size of 0.7 $\mu$m. GF filters are formed of borosilicate glass fibre. In some embodiments, the filter comprises at least a grade D and a grade F filter. Other suitable filters will be known to the skilled person.

In embodiments, the maximum thickness of the filter as measured in the direction of sample flow through the filter may be from 1000 $\mu$m to 2000 $\mu$m. In some embodiments, the maximum thickness of the filter device as measured in the direction of sample flow is from 1500$\mu$m to 2000$\mu$m. The first porous region may have a maximum thickness (relative to the direction of fluid flow) of from 500 to 600$\mu$m. The second porous region may have a maximum thickness (relative to the direction of fluid flow) of from 250 to 480$\mu$m. In embodiments, the first porous region has a maximum thickness (relative to the direction of fluid flow) from 500 to 600$\mu$m and the second porous region has a maximum thickness (relative to the direction of fluid flow) of from 250 to 480$\mu$m. It will be appreciated that such porous regions will typically have a uniform thickness (relative to the direction of fluid flow) across the width of the region. This has the advantage of ensuring a consistent flow path length for all parts of the fluid through the filter.

By thickness, this will, unless otherwise stated to the contrary, be understood to refer to the length of the porous region in the direction of fluid flow.

It will be appreciated that the filter may typically comprise at least two opposing faces, a first face being arranged to be contacted in use by the fluid sample first, following which the fluid sample passes through the filter via the second face (wherein at least some of the sample is retained on the filter).

The first porous region is nearer to the first face than the second face. In some embodiments, the first face comprises the first porous region. In some embodiments, the second face may comprise the second porous region. The second region may on the other hand be positioned within the main body of the filter such that the second face may comprise a further (e.g. third) region of different nominal porosity compared to the second region, e.g. it may have the same porosity as the first porous region. In such embodiments the second porous region is located between the first and further (e.g. third) porous regions.

The first porous region may be integral with (e.g. formed with), or attached to, the second region within the same filter. Typically and conveniently, the first porous region may correspond to a distinct filter having the given (larger) nominal pore size (i.e. a first filter) and the second porous region may correspond to a second distinct filter having the second (smaller) nominal pore size, i.e. wherein the first filter is upstream relative to the fluid flow through the device relative to the second filter which is downstream relative to the fluid flow through the device. The distinct filters may be bonded together, or may not be bonded together (e.g. they may be simply in separable contact, e.g. mutually stacked with respect to the direction of fluid flow). The combination of distinct filters or porous regions may be provided in a stacked arrangement relative to the flow of fluid through the filter. For a gravity column this will usually be a vertically stacked arrangement but for some apparatus, such as a spin apparatus, it will be appreciated that the flow of fluid through the filter may not be vertical. In the case of fluid flow in a spin apparatus, this need not be vertical due to the centrifugal force applied and so in such arrangements the stacking in this context need not be vertical in relationship provided that the first filter or region is upstream relative to the fluid flow through the device relative to the second filter or region, which is downstream relative to the fluid flow through the device.

In some embodiments the filter comprises (or in particular embodiments consists of) a first porous region and a second porous region, the first porous region being arranged to be contacted in use by the sample before the second porous region, wherein the nominal pore size of the first porous region is from 2 $\mu$m to 3 $\mu$m and the nominal pore size of the second porous region is from 0.5 to 1.8 $\mu$m, the filter comprising a silicate or silica-based material, optionally borosilicate glass (preferably borosilicate glass fibres).

In some embodiments the filter comprises (or in particular embodiments consists of) a first porous region and a second porous region, the first porous region being arranged to be contacted in use by the sample before the second porous region, wherein the nominal pore size of the first porous region is from 2 $\mu$m to 3 $\mu$m and the nominal pore size of the second porous region is from 0.5 to 1.8 $\mu$m and the maximum thickness of the filter as measured in the direction of sample flow through the filter is from 1000$\mu$m to 2000$\mu$m, optionally no more than 1500$\mu$m.

In some embodiments the filter comprises (or in particular embodiments consists of) a first porous region and a second porous region, the first porous region being arranged to be contacted in use by the sample before the second porous region, the nominal pore size of the second region being around 0.7$\mu$m (e.g. 0.7 $\mu$m) and a nominal pore size of the first region being around 2.7 μm (e.g. 2.7 μm),and the maximum thickness of the filter as measured in the direction of sample flow through the filter is from 1000μm to 2000μm, optionally no more than 1500μm.

In some embodiments the filter comprises (or in particular embodiments consists of) a first porous region and a second porous region, the first porous region being arranged to be contacted in use by the sample before the second porous region, wherein the nominal pore size of the first porous region is from 2 μm to 3 μm and the nominal pore size of the second porous region is from 0.5 to 1.8 μm, the maximum thickness of the filter as measured in the direction of sample flow through the filter is from 1000μm to 2000μm, optionally no more than 1500μm and wherein the filter comprises a silicate or silica-based material, optionally borosilicate glass (preferably borosilicate glass fibres).

The filter may comprise (or in particular embodiments consist of) a plurality of first porous regions and a second porous region, the first porous regions being arranged to be contacted in use by the sample before the second porous region, wherein the nominal pore size of each first porous region is from 2 μm to 3 μm and the nominal pore size of the second porous region is from 0.5 to 1.8 μm.

In some embodiments the filter comprises (or in particular embodiments consists of) a plurality of first porous regions and a second porous region, the first porous regions being arranged to be contacted in use by the sample before the second porous region, wherein the nominal pore size of each first porous region is from 2 μm to 3 μm and the nominal pore size of the second porous region is from 0.5 to 1.8 μm and the filter comprises a silicate or silica-based material, optionally borosilicate glass (preferably borosilicate glass fibres).

In some embodiments the filter comprises (or in particular embodiments consists of) a plurality of first porous regions and a second porous region, the first porous regions being arranged to be contacted in use by the sample before the second porous region, wherein the nominal pore size of each first porous region is around 2.7 μm (e.g. 2.7 μm) and the nominal pore size of the second porous region is around 0.7μm (e.g. 0.7 μm).

In some embodiments the filter comprises (or in particular embodiments consists of) a plurality of first porous regions and a second porous region, the first porous regions being arranged to be contacted in use by the sample before the second porous region, wherein the nominal pore size of each first porous region is around 2.7 μm (e.g. 2.7 μm) and the nominal pore size of the second porous region is around 0.7μm (e.g. 0.7 μm), the filter comprising a silicate or silica-based material, optionally borosilicate glass (preferably borosilicate glass fibres).

The filter may comprise (or in particular embodiments consist of) a plurality of first porous regions and a second porous region, the first porous regions being arranged to be contacted in use by the sample before the second porous region, wherein the nominal pore size of each first porous region is from 2 μm to 3 μm and the nominal pore size of the second porous region is from 0.5 to 1.8 μm, and the maximum thickness of the filter as measured in the direction of sample flow through the filter is from 1000μm to 2000μm.

In some embodiments the filter comprises (or in particular embodiments consists of) a plurality of first porous regions and a second porous region, the first porous regions being arranged to be contacted in use by the sample before the second porous region, wherein the nominal pore size of each first porous region is around 2.7 μm (e.g. 2.7 μm) and the nominal pore size of the second porous region is around 0.7μm (e.g. 0.7 μm), and the maximum thickness of the filter as measured in the direction of sample flow through the filter is from 1000μm to 2000μm.

The filter may comprise (or in particular embodiments consist of) a plurality of first porous regions and a second porous region, the first porous regions being arranged to be contacted in use by the sample before the second porous region, wherein the nominal pore size of each first porous region is from 2 μm to 3 μm and the nominal pore size of the second porous region is from 0.5 to 1.8 μm, and the maximum thickness of the filter as measured in the direction of sample flow through the filter is from 1500μm to 2000μm.

The filter may comprise (or in particular embodiments consist of) a plurality of first porous regions and a second porous region, the first porous regions being arranged to be contacted in use by the sample before the second porous region, wherein the nominal pore size of each first porous region is from 2 μm to 3 μm and the nominal pore size of the second porous region is from 0.5 to 1.8 μm, the maximum thickness of the filter as measured in the direction of sample flow through the filter is from 1500μm to 2000μm and the filter comprises a silicate or silica-based material, optionally borosilicate glass (preferably borosilicate glass fibres).

In some embodiments the filter comprises (or in particular embodiments consists of) a plurality of first porous regions and a second porous region, the first porous regions being arranged to be contacted in use by the sample before the second porous region, wherein the nominal pore size of each first porous region is around 2.7 μm (e.g. 2.7 μm) and the nominal pore size of the second porous region is around 0.7μm (e.g. 0.7 μm), and the maximum thickness of the filter as measured in the direction of sample flow through the filter is from 1500μm to 2000μm.

The filter may comprise (or in particular embodiments consist of) a plurality of first porous regions and a second porous region, the first porous regions being arranged being arranged to be contacted in use by the sample before the second porous region, wherein the nominal pore size of each first porous region is from 2 μm to 3 μm and the nominal pore size of the second porous region is from 0.5 to 1.8 μm, the maximum thickness of the filter as measured in the direction of sample flow through the filter is from 1000μm to 2000μm and the filter comprises a silicate or silica-based material, optionally borosilicate glass (preferably borosilicate glass fibres).

In some embodiments the filter comprises (or in particular embodiments consists of) a plurality of first porous regions and a second porous region, the first porous regions being arranged to be contacted in use by the sample before the second porous region, wherein the nominal pore size of each first porous region is around 2.7 $\mu$m (e.g. 2.7 $\mu$m) and the nominal pore size of the second porous region is around 0.7$\mu$m (e.g. 0.7 $\mu$m), and the maximum thickness of the filter as measured in the direction of sample flow through the filter is from 1500$\mu$m to 2000$\mu$m, the filter comprising a silicate or silica-based material, optionally borosilicate glass (preferably borosilicate glass fibres).

The plurality of first porous regions may comprise (or in particular embodiments consists of) two, three, four, five or six first porous regions. In some embodiments the plurality of first porous regions comprises (or in particular embodiments consists of) two first porous regions.

The filter may comprise (or in particular embodiments consist of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m.

In some embodiments the filter comprises (or in particular embodiments consists of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m).

The filter may comprise (or in particular embodiments consist of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m, the maximum thickness of the filter as measured in the direction of sample flow through the filter is from 1000$\mu$m to 2000$\mu$m.

In some embodiments the filter comprises (or in particular embodiments consists of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m), wherein the maximum thickness of the filter as measured in the direction of sample flow through the filter is from 1000$\mu$m to 2000$\mu$m.

The filter may comprise (or in particular embodiments consist of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m, the maximum thickness of the filter as measured in the direction of sample flow through the filter is from 1500$\mu$m to 2000$\mu$m.

In some embodiments the filter comprises (or in particular embodiments consists of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m), wherein the maximum thickness of the filter as measured in the direction of sample flow through the filter is from 1500$\mu$m to 2000$\mu$m.

The filter may comprise (or in particular embodiments consist of) a first porous region, a second porous region and a third porous region, the first porous region being arranged being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m, and the filter comprises a silicate or silica-based material, optionally borosilicate glass (preferably borosilicate glass fibres).

In some embodiments the filter comprises (or in particular embodiments consists of) a first porous region, a second porous region and a third porous region, the first porous region being arranged being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m) and the filter comprises a silicate or silica-based material, optionally borosilicate glass (preferably borosilicate glass fibres).

The filter may comprise (or in particular embodiments consist of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m, the maximum thickness of the filter as measured in the direction of sample flow through the filter is from 1000$\mu$m to 2000$\mu$m and the filter comprises a silicate or silica-based material, optionally borosilicate glass (preferably borosilicate glass fibres).

In some embodiments the filter comprises (or in particular embodiments consists of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m), wherein the maximum thickness of the filter as measured in the direction of sample flow through the filter is from 1000$\mu$m to 2000$\mu$m and the filter comprises a silicate or silica-based material, optionally borosilicate glass (preferably borosilicate glass fibres).

The filter may comprise (or in particular embodiments consist of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is from 2 $\mu$m to 3 $\mu$m and the nominal pore size of the second porous region is from 0.5 to 1.8 $\mu$m, the maximum thickness of the filter as measured in the direction of sample flow through the filter is from 1500$\mu$m to 2000$\mu$m and the filter comprises a silicate or silica-based material, optionally borosilicate glass (preferably borosilicate glass fibres).

In some embodiments the filter comprises (or in particular embodiments consists of) a first porous region, a second porous region and a third porous region, the first porous region being arranged to be contacted in use by the sample before the second porous region and the third porous region being arranged to be contacted in use by the sample after the second porous region, wherein the nominal pore size of the first and third porous region is around 2.7 $\mu$m (e.g. 2.7 $\mu$m) and the nominal pore size of the second porous region is around 0.7$\mu$m (e.g. 0.7 $\mu$m), wherein the maximum thickness of the filter as measured in the direction of sample flow through the filter is from 1500$\mu$m to 2000$\mu$m and the filter comprises a silicate or silica-based material, optionally borosilicate glass (preferably borosilicate glass fibres).

The filters of the invention are eminently suitable for use in nucleic acid extraction columns. Conveniently, the filter device may thus be provided in a nucleic acid extraction column, such as is known to the skilled person in the art, e.g. a spin column as described in patent application number WO 2016/147004. The filter in the spin column is preferably provided in the form of at least one sheet or membrane comprising borosilicate fibres.

Thus, according to a second aspect of the invention, there is provided a nucleic acid extraction column for isolating nucleic acid from a sample, the nucleic acid extraction column comprising the filter according to the first aspect or any embodiment thereof described herein.

Typically, the nucleic acid extraction column is in the form of a tube which is suitable for insertion into a microtube, for example an Eppendorf tube. The filter may form the base of the column. In use, a sample may be added to the column such that the sample flows through and/or is retained by the filter. Any sample which flows through the filter can then be retained by the microtube.

It will be appreciated that the body of the nucleic acid extraction column, excluding the filter, may be formed of plastic. Other suitable materials can be envisaged by the skilled person.

According to a third aspect of the invention, there is provided a method of isolating nucleic acid from a sample using a filter according to the first aspect of the invention. Put another way, the present invention also provides the use of a filter according to the first aspect of the invention in a method of isolating nucleic acid from a sample. In embodiments, the method comprises:

(i) contacting a sample (e.g. a lysed sample) comprising nucleic acid with an alcohol and a salt (optionally a solution comprising the alcohol and salt) (i.e. to provide a composition comprising the nucleic acid, alcohol and salt), the salt optionally provided in solution at a concentration of 0.05 to 10M; and

(ii) contacting a filter according to the first aspect of the invention described herein with the composition comprising nucleic acid obtained following step (i) to provide a filter having nucleic acid bonded thereto; and

(iii) preferably, treating the filter having the nucleic acid bonded thereto with a wash solution comprising alcohol and optionally a salt; and

(iv) eluting at least a portion of the nucleic acid from the filter.

The method may optionally further comprise: (v) carrying out a second elution of nucleic acid from the filter following step (iv), e.g. wherein elution comprises incubating the filter in an elution buffer at a temperature not exceeding 90°C, optionally followed by centrifugation of the filter.

The above method provides an improved method for the isolation of nucleic acid fragments. In particular, the nucleic acid isolated obtained using filters of the invention is generally purer and more intact compared to corresponding methods which do not use filters of the invention (see examples and corresponding figures). In addition, the yield of total nucleic acid fragments, and the ratio of long to short nucleic acid fragments obtained, is improved (e.g. Figure 1C).

In some embodiments the sample comprises purified nucleic acid. In other embodiments the sample comprises or is a lysed biological sample.

In some embodiments the method comprises a step of lysing a biological sample comprising nucleic acid. The lysing may be performed prior to the contacting step (i) described above or simultaneously with the contacting step (i) above. When performed prior to step (i) above, the lysed biological sample may then form the sample referred to in step (i). When the lysing is performed simultaenously with step (i), the resulting composition comprising the nucleic acid will be a lysed composition which may then be subject to step (ii).

Skilled artisans will be aware of suitable lysis conditions for releasing nucleic acids from biological material in a sample prior to filtration according to the invention. Typically, lysis is caused by treating a biological sample with one or more of a salt, a surfactant, a chelating agent, and / or an enzyme. Such lysis components can thus be added to the sample containing nucleic acid prior to the alcohol and / or salt of step (i), or may be added as part of the same mixture as the alcohol and / or salt of step (i). The resulting composition formed following lysis may be cleaned up (e.g. to remove unwanted biological components) prior to submitting the composition to step (i) of the method. The resulting lysis composition may alternatively be submitted to step (i) without any clean up (in which case the resulting composition may be the composition containing nucleic acid for submission to step (ii) of the method directly). If lysis is performed as part of step (i) then it will be appreciated that the resulting lysed sample will be a lysed composition which may be then submit to step (ii) of the method directly.

In embodiments comprising a step of lysing a biological sample, the lysing may comprise lysing biological material in the sample with an aqueous solution to produce an aqueous lysed composition to form the sample of step (i). The aqueous solution (used for lysis) may comprise a surface active agent. The aqueous solution may additionally or alternatively comprise a proteinase, for example proteinase K and preferably a chelating agent. In some embodiments the solution may contain a salt. The lysing step may thus additionally or alternatively comprise adding an aqueous solution comprising a salt. The salt may be an inorganic salt or organic salt. The salt may for instance be a metal salt, such as an alkali metal salt, for example a lithium salt. The respective salt may be provided with any suitable counter ion such as a halide, e.g. a metal halide, e.g. lithium halide, preferably lithium chloride. The salt may be provided in the form of a solution, e.g. provided in the form of a solution of from 0.05 to 10M, such as 0.5 to 1.0M, e.g. around 0.8M). The salt may be provided so as to provide a concentration in the composition obtained following step (i) of from 0.1M to 0.5M, e.g. 0.2M to 0.3M, e.g. 0.23M. In some embodiments the aqueous solution comprises an alcohol. The alcohol may comprise a $C_{1-3}$ alkanol, for example ethanol or iso-propanol. The $C_{1-3}$ alkanol may comprise at least 75%, optionally at least 95% ethanol or iso-propanol. In some embodiments the aqueous solution comprises a salt, for example lithium chloride.

The biological sample (e.g. cells) may be thoroughly admixed with the aqueous solution (e.g. by pipetting) and lysis effected at ambient temperature for a suitable period of time. A time period of 15 to 25 minutes, e.g. about 20 minutes is generally suitable. In some embodiments, lysis may be effected at a temperature of 40 to 60°C. For example, lysis may be effected at this temperature in embodiments wherein the aqueous solution comprises proteinase K. Lysis results in the formation of a lysed composition.

The lysed composition may be centrifuged to form a pellet. The pellet may be re-suspended in a solution to form the sample of step (i).

The chelating agent, which is most preferably EDTA or a salt thereof such as an alkali metal (e.g. sodium) salt, may be provided as an aqueous solution, e.g. wherein the concentration of EDTA or salt thereof in the solution is from 1 to 20 mM per litre, more preferably 8 to 15 millimoles per litre, more preferably 9 to 11 millimoles per litre and most preferably about 10 millimoles per litre. Preferably, the amount of chelating agent is such that the resulting composition comprising nucleic acid (e.g. the composition which is then contacted with the filter in step (ii) contains from 0.5-5 mmol EDTA, such as 2-4 mmol, e.g. around 2.85mmol.

The surface active agent may be a non-ionic surfactant but is more preferably an anionic surfactant, most preferably SDS (Sodium dodecyl sulfate). The aqueous solution may comprise the surfactant in an amount of from 0.1 to 1.5% by weight, preferably from about 0.2 to 0.8% by weight, most preferably about 0.5% by weight. Preferably, the amount of surfactant is such that the resulting composition comprising nucleic acid (e.g. the composition which is then contacted with the filter in step (ii) contains from 0.05-0.5 % w/v surfactant (e.g. SDS), such as from 0.1-0.2% w/v surfactant (e.g. SDS), e.g. around 0.14 % w/v).

The aqueous solution may have a pH of 7 to 8 but in embodiments is not necessarily buffered at a pH in this range. Thus, the aqueous solution may be an unbuffered composition.

In certain embodiments (e.g. for the lysis of gram negative bacteria), lysis may be effected without treatment of the cells with a protease enzyme (e.g. Proteinase K) and without the need for a heating step. In other embodiments (e.g. for treatment of tissue such as blood for the purpose of lysing whole blood cells) the sample may be treated with a protease enzyme prior to step (i) (e.g. Proteinase K). In the latter embodiments, treatment with the protease enzyme (Proteinase K) may be effective simultaneously with treatment with a lysis solution comprising a salt, e.g. lithium salt.

In an exemplary lysing step, a biological sample containing nucleic acids (e.g. a cell sample) is contacted with a lysis solution comprising a metal salt (preferably LiCI, more preferably in a concentration of around 0.8M) a chelating agent (preferably SDS, such as in a concentration of around 0.5% w/v), an enzyme (preferably a protease, such as proteinase K) and a chelating agent (preferably EDTA, e.g. in a concentration in the solution of around 10mM) and the composition mixed to lyse the sample. The resulting sample may then be optionally centrifuged to separate nucleic acid from other biological materials. The sample containing nucleic acids obtained following lysis may then be contacted with an alcohol and salt according to step (i). The alcohol and salt may be supplied simultaneously or sequentially.

In step (i), the alcohol and salt may be contacted with nucleic acid simultaneously or sequentially. For instance, the salt may be added in the form of a salt solution followed by addition of the alcohol, or vice versa. Alternatively, the salt and alcohol may be provided as components within the same composition (e.g. solution) and the composition either added to the nucleic acid or vice versa. If a lysis step is performed, the salt and alcohol may already be present following the lysis step, e.g. by virtue of being components of the lysis mixture.

The salt according to step (i) may be any suitable inorganic or organic salt. The salt may comprise (or in embodiments may be) a chaotropic agent. The chaotropic agent preferably includes or is a guanidinium salt (e.g. guanidinium halide, e.g. chloride, also known as guanidinium hydrochloride or guanidine hydrochloride, referred to elsewhere herein as "GuHCI"). The guanidinium salt may be provided so as to have a concentration in the composition containing nucleic acid, alcohol and salt (obtained following step (i)) in the range of 0.05 to 10M, preferably 0.1 to 3M, preferably 0.1 to 2M, more preferably 0.5 to 0.9M, and most preferably 0.6 to 0.7M in the solution, most preferably 0.67M. By way of example, the salt (e.g. guanidinium salt) may be added as a solution having a concentration that gives rise to the above concentrations once added to the sample containing nucleic acid. For example, the salt (e.g. guanidinium salt) may be provided as a 1-5M solution, e.g. around 2M. This is typically provided as an alcohol solution as described below.

The chaotropic agent may be dissolved in a $C_{1-3}$ alkanol, for example ethanol or iso-propanol. The $C_{1-3}$ alkanol may comprise at least 75% v/v, optionally at least 95% v/v ethanol or iso-propanol.

For the avoidance of doubt, it will be understood that reference to a percentage of alcohol, for example 96% ethanol, refers to an aqueous liquid composition containing 96% v/v ethanol (e.g. and up to 4% v/v water).

The alcohol provided in step (i) may comprise (or may be) a $C_{1-3}$ alkanol, for example isopropanol or ethanol. The $C_{1-3}$alkanol may be provided in an amount such that the composition comprising nucleic acid obtained from step (i), i.e. submitted to step (ii) contains at least 10%, at least 20%, at least 30%, or at least 40% v/v alcohol. Typically the alcohol content of the composition formed in step (i) does not exceed about 60% v/v alcohol. In preferred embodiments, the alcohol concentration in the composition formed following step (i) and submitted to step (ii) does not exceed about 35% v/v, e.g. does not exceed around 30% v/v. The $C_{1-3}$alkanol may be provided in an amount such that the composition comprising nucleic acid formed from step (i), i.e. submitted to step (ii) contains from 25 to 40% by volume, optionally from 25 to 30% by volume.

In the above method, the method preferably (but need not necessarily) comprises a wash step (iii). That is, the method comprises steps (i), (ii) and (iv) and preferably also comprises the wash step (iii). Skilled artisans will appreciate that the wash step (iii) will assist in providing purer nucleic acid material compared to if the wash step were omitted. It is thus desirable to include wash step (iii). The wash solution will typically contain an alcohol, which may be an alcohol as described above. In some embodiments the wash solution further comprises a salt. The salt is preferably a lithium or guanidinium halide, for example lithium chloride or guanidinium chloride. Treatment with the wash solution is intended to remove impurities, to leave substantially pure nucleic acid immobilised on the filter. The filter is then separated from the liquid, optionally by centrifugation.

The salt may be present in the wash solution at a concentration of 0.05 to less than 10 moles per litre, preferably 0.05 to less than 1 moles per litre, preferably 0.1 to 0.9 moles per litre, more preferably 0.6 to 0.9 moles per litre, even more preferably 0.75 to 0.85 moles per litre, and most preferably about 0.8 moles per litre. In some embodiments the wash solution comprises the salt at a concentration of 0.2 to 0.9M.

The filter may be treated with a plurality of wash solutions. A first wash solution may comprise an alcohol and a salt (e.g. lithium or guanidinium halide, for example lithium chloride or guanidinium chloride), optionally at a concentration of 0.05 to 10M. It will be appreciated that the filter may be separated from the liquid, optionally by centrifugation, after each wash.

In embodiments comprising a second wash solution, the second wash liquid may contain an alcohol. In some embodiments the second wash liquid comprises an alcohol and a salt (e.g. lithium or guanidinium halide, for example lithium chloride or guanidinium chloride) and these components may be provided at a different salt concentration to the first wash solution. For instance, if the first wash solution has a salt concentration of 0.05 to 1M, then in embodiments the second wash may be conducted at a concentration which is not from 0.05 to 1M, or which is lower or higher than the first wash solution.

The alcohol may comprise a $C_{1-3}$ alkanol, for example isopropanol or ethanol. The $C_{1-3}$ alkanol may be at least 30%, at least 50%, preferably at least 70% v/v. In some embodiments the $C_{1-3}$ alkanol comprises 75% to 100% v/v of ethanol. In some embodiments the $C_{1-3}$ alkanol comprises 90% v/v ethanol.

The method of the third aspect of the invention may be used for isolating nucleic acids such as DNA (particularly genomic DNA) from a wide range of nucleic acid-containing samples of biological origin. The sample from which the nucleic acid (e.g. DNA) is to be isolated may be, or may comprise, cells, for example animal (including mammalian) cells, plant cells, bacterial cells, or yeast or other fungal cells. Alternatively, the sample from which the nucleic acid (e.g. DNA) is to be isolated may be, or may comprise, one or more viruses. The cells, virus(es) or other nucleic acid-containing sample from which the nucleic acids (e.g. comprising DNA) are to be isolated may originate from or have been present in a tissue sample or body fluids such as blood, sputum, urine or CSF. The sample may be an isolated fluid sample derived from a biological sample. For example, a biological sample such as cells may be centrifuged to form a cell pellet. The resulting pellet and/or supernatant may be isolated and re-suspended to form the sample for use in the present method. In some embodiments the sample comprises purified nucleic acid. In other embodiments the sample comprises a lysed biological sample from a nucleic acid-containing sample of biological origin, as described herein. The nucleic acids, for example DNA, obtained by the method of the invention are sufficiently pure to be used for the purpose of further analysis by techniques well known in the art. The nucleic acid, e.g. DNA, may be used, for example, for the purposes of research, or for diagnosis of a medical condition, as required.

It will be appreciated that where the filter is contacted with a liquid and/or solution, the liquid and/or solution may flow through the filter. Flow may be effected, for example by spin methods, such as centrifugation, or by gravity. The liquid and/or solution can then be separated from the filter between steps. In this way, nucleic acid can be retained on the filter while the remaining liquid flows through the filter, until step (iv) when the nucleic acid is isolated from the filter. During fluid flow, the nucleic acids (that are ultimately to be isolated) become bound to the filter device and the majority of impurities (proteins etc.) pass through the filter in the liquid which may then be collected and discarded.

By eluting nucleic acid from the filter, this will be understood to refer to the transfer of nucleic acid bound to the filter to an eluent. As the skilled person will appreciated, eluent refers to a solution containing the isolated nucleic acid. Hence, the elution comprises contacting the filter with an elution buffer.

The elution buffer may comprise (consists essentially of or consists of) a buffered solution of EDTA or a salt thereof. A particular suitable elution solution comprises Tris-HCl in a concentration of about 10mM, and EDTA disodium salt dihydrate at a concentration of about 0.5mM, the solution having a pH of 8 to 9. Another suitable elution solution may comprise DNase and RNase-free water.

In some embodiments, step (iv) comprises incubating the filter in an elution buffer at a temperature from room temperature to 100°C, optionally at a temperature from 65 to 90°C, optionally 70 to 90°C. Without wishing to be bound by theory, the inventor believes that a temperature higher than room temperature improves the ratio of long to short DNA obtained and/or the yield of DNA obtained. The examples demonstrate particularly advantageous results when temperatures of 70 °C or above are used. In some embodiments step (iv) comprises incubating the filter in an elution buffer at a temperature of 80°C. In some embodiments step (iv) comprises incubating the filter in an elution buffer at a temperature of 65°C. In the context of the present invention, room temperature will be understood to be a temperature of 10°C to 20°C. Incubation in step (iv) may be for a time period from 30 seconds to 2 minutes, optionally 1 minute.

The nucleic acid may be eluted from the filter into the elution buffer by centrifugation, such that the centrifugal force transfers the nucleic acid from the filter to the elution buffer. As the elution buffer flows through the filter to capture the nucleic acid, this then forms the eluate.

In some embodiments the nucleic acid is eluted from the filter into the elution buffer by two centrifugation steps. In such embodiments it will be appreciated that elution buffer is applied to the filter, and then flows through, twice. The eluent may comprise the second elution buffer once centrifuged (and thus containing isolated nucleic acid).

Centrifugation may be at a speed so as to provide a g-force of from 600 to 8000g. In step (iv), centrifugation may be conducted at a speed corresponding to 600 to 2000g, optionally 1000 to 2000g, for example 1180g. Centrifugation may be effected at a speed corresponding to at least 2600g and no more than 8000g. In some embodiments, centrifugation may be effected at a speed corresponding to 4000 to 5000g, optionally 4722g.

The capability of the present method to generate a high ratio of large to small nucleic acid fragments is especially surprising considering that any centrifugation steps can be effected at a high g force without any detrimental effect to this ratio.

The filter may be provided as a component of a nucleic acid extraction column, as defined herein.

In the case where the method of the invention is being practised with a nucleic acid extraction column, optionally a spin column, containing a filter on which the nucleic acids is immobilised, the elution buffer may be added to the nucleic acid extraction column which is then centrifuged to cause the elution buffer to elute the nucleic acids from the filter for collection.

The collected nucleic acids may then be further analysed or processed as required.

In embodiments, the method further comprises a sequencing step following isolation of the nucleic acids following step (iv).

According to a fourth aspect of the present invention, there is provided a method of isolating nucleic acid from a sample, the method comprising:

(i) contacting a sample comprising nucleic acid with an alcohol and a salt (optionally a solution comprising the alcohol and salt) (i.e. to form a composition containing the nucleic acid, alcohol and salt), the salt optionally provided in solution at a concentration of 0.05 to 10M; and

(ii) contacting a filter with the composition comprising nucleic acid obtained following step (i) to provide a filter having nucleic acid bonded thereto; and

(iii) preferably, treating the filter having the nucleic acid bonded thereto with a wash solution comprising alcohol; and

(iv) eluting at least a portion of the nucleic acid from the filter, wherein elution comprises incubating the filter in an elution buffer at a temperature of from 70 to 90°C.

The method may in embodiments further comprise: (v) carrying out a second elution of nucleic acid from the filter, e.g. wherein elution comprises incubating the filter in an elution buffer at a temperature from 10 to 90°C, optionally followed by centrifugation of the filter.

It will be appreciated that the respective method conditions and component definitions of embodiments of the third aspect, as described herein are also applicable to the fourth aspect and the fifth aspects of the invention, as described below.

A lysis step may thus be provided prior to step (i) or simultaneously with step (i) as described according to the third aspect of the invention. Any definition of the lysis step, or embodiments containing a lysis step as described herein may thus apply to the fourth aspect of the invention.

In step (i), the alcohol and salt may be contacted with nucleic acid simultaneously or sequentially. For instance, the salt may be added in the form of a salt solution followed by addition of the alcohol, or vice versa. Alternatively, the salt and alcohol may be provided as components within the same composition (e.g. solution) and the composition either added to the nucleic acid or vice versa. If a lysis step is performed, the salt and alcohol may already be present following the lysis step, e.g. by virtue of being components of the lysis step.

The salt provided in step (i) may be any suitable inorganic or organic salt. The salt may comprise (or in embodiments may be) a chaotropic agent. The chaotropic agent preferably includes, or is, a guanidinium salt (e.g. guanidinium halide, e.g. chloride, also known as guanidinium hydrochloride or guanidine hydrochloride). The guanidinium salt may be provided so as to have a concentration in the composition containing nucleic acid, alcohol and salt (obtained following step (i)) in the range of 0.05 to 10M, preferably 0.1 to 3M, preferably 0.1 to 2M, more preferably 0.5 to 0.9M, and most preferably 0.6 to 0.7M in the solution. By way of example, the salt (e.g. guanidinium salt) may be added as a solution having a concentration that gives rise to the above concentrations once added to the sample containing nucleic acid. For example, the salt (e.g. guanidinium salt may be provided as a 1-5M solution, e.g. around 2M). This is typically provided as an alcohol solution as described below.

The chaotropic agent may be dissolved in a $C_{1-3}$ alkanol, for example ethanol or iso-propanol. The $C_{1-3}$ alkanol may comprise at least 75%, optionally at least 95% ethanol or iso-propanol.

The alcohol provided in step (i) may comprise (or may be) a $C_{1-3}$ alkanol, for example iso-propanol or ethanol. The $C_{1-3}$ alkanol may be provided in an amount such that the composition comprising nucleic acid formed from step (i), i.e. submitted to step (ii) contains at least 10%, at least 20%, at least 30%, or at least 40% v/v alcohol. Typically the alcohol content of the composition formed in step (i) does not exceed about 60% v/v alcohol. The $C_{1-3}$ alkanol may be provided in an amount such that the composition comprising nucleic acid formed from step (i), i.e. submitted to step (ii) contains from 25 to 40% by volume, optionally from 25 to 30% by volume, e.g. 28% v/v.

In the above method, the method preferably (but need not necessarily) comprises a wash step (iii). That is, the method comprises steps (i), (ii) and (iv) and preferably also comprises the wash step (iii). Skilled artisans will appreciate that the wash step (iii) will assist in providing purer nucleic acid material compared to if the wash step were omitted. It is thus desirable to include wash step (iii). The wash solution will typically contain an alcohol, which may be an alcohol as described above. The wash solution may further comprise a salt. The salt may be as defined in relation to the third aspect. For instance, the salt is preferably a lithium or guanidinium halide, for example lithium chloride or guanidinium chloride. Treatment with the wash solution is intended to remove impurities, to leave substantially pure nucleic acid immobilised on the filter. The filter is then separated from the liquid, optionally by centrifugation. The salt may be present in the wash solution at a concentration of 0.05 to less than 10 moles per litre, preferably 0.05 to less than 1 moles per litre, preferably 0.1 to 0.9 moles per litre, more preferably 0.6 to 0.9 moles per litre, even more preferably 0.75 to 0.85 moles per litre, and most preferably about 0.8 moles per litre. In some embodiments the wash solution comprises the salt at a concentration of 0.2 to 0.9M.

The filter may be treated with a plurality of wash solutions. A first wash solution may comprise an alcohol and optionally also a salt (e.g. lithium or guanidinium halide, for example lithium chloride or guanidinium chloride), optionally at a concentration of 0.05 to 10M. It will be appreciated that the filter may be separated from the liquid, optionally by centrifugation, after each wash.

In embodiments comprising a second wash solution, the second wash liquid may contain an alcohol. In some embodiments the second wash liquid comprises an alcohol and a salt (e.g. lithium or guanidinium halide, for example lithium chloride or guanidinium chloride) and these components may be provided at a different salt concentration to the first wash solution. For instance, if the first wash solution has a salt concentration of 0.05 to 1M, then in embodiments the second wash may be conducted at a concentration which is not from 0.05 to 1M, or which is lower or higher than the first wash solution.

The alcohol in the wash solution may comprise a $C_{1-3}$ alkanol, for example isopropanol or ethanol. The $C_{1-3}$ alkanol may be at least 30%, at least 50%, preferably at least 70% v/v in solution. In some embodiments the $C_{1-3}$ alkanol comprises 75% to 100% v/v of ethanol. In some embodiments the $C_{1-3}$ alkanol comprises 90% v/v ethanol.

The elution buffer may comprise (or consist essentially of, or consist of) a buffered solution of EDTA or a salt thereof. A particular suitable elution solution comprises Tris-HCl in a concentration of about 10mM, and EDTA disodium salt dihydrate at a concentration of about 0.5mM, the solution having a pH of 8 to 9. Another suitable elution solution comprises DNase and RNase-free water.

In some embodiments the incubation temperature of step (iv) is from 75 to 85°C, optionally 80°C. It has been discovered that 80 °C is particularly advantageous. Incubation in step (iv) may be for a time period from 30 seconds to 2 minutes, optionally 1 minute.

The nucleic acid may be eluted from the filter into the elution buffer by centrifugation, such that the centrifugal forces transfer the nucleic acid from the filter to the elution buffer. As the elution buffer flows through the filter to capture the nucleic acid, this then forms the eluent.

In some embodiments the nucleic acid is eluted from the filter into the elution buffer by two centrifugation steps. In such embodiments it will be appreciated that elution buffer is applied to the filter, and then flows through, twice. The eluent may comprise the second elution buffer once centrifuged (and thus containing isolated nucleic acid).

Centrifugation may be at a speed corresponding to a g-force of from 600 to 8000g. In step (iii), centrifugation may be at a speed corresponding to 600 to 2000 g, optionally from 1000 to 2000g, for example around 1180g. Centrifugation may be effected at a speed corresponding to at least 2000 and no more than 8000g. In some embodiments, centrifugation may be effected at a speed corresponding to 4000 to 5000g, optionally around 4722g

The filter may be provided as a component of a nucleic acid extraction column, as defined herein.

The filter as used for the method of the above aspect may be a silica or silica-based material. The silica or silica-based material may be a glass, preferably a borosilicate glass. The filter may comprise a fibrous material, and preferably comprises fibres of a silica or silica-based material. The filter preferably comprises borosilicate glass fibres. For preference, the filter is in the form of a fibrous sheet or membrane in which the fibres are of a borosilicate glass. In preferred embodiments, the filter (i.e. as provided in step (ii)) may be any filter as described according to the first aspect of the invention, or any embodiments thereof.

In the case where the method of the invention is being practised with a nucleic acid extraction column, optionally a spin column, comprising a filter on which the nucleic acids are immobilised, the elution buffer may be added to the nucleic acid extraction column which is then centrifuged. In preferred embodiments, the column may be a column as described according to the second aspect of the invention, or embodiments thereof.

In embodiments, the method further comprises a sequencing step following isolation of the nucleic acids obtained from step (iv).

According to a fifth aspect of the present invention, there is provided a method of purifying nucleic acid isolated from a sample, the method comprising:

(i) contacting the nucleic acid with an alcohol and a salt (optionally a solution comprising the alcohol and salt) (i.e. to provide a composition containing the nucleic acid, alcohol and salt), the salt optionally provided in a solution at a concentration of 0.05 to 10M;

(ii) contacting a filter with the composition comprising the nucleic acid obtained following step (i) to provide a filter having nucleic acid bonded thereto;

(iii) preferably, treating the filter having the nucleic acid bonded thereto with a wash solution comprising alcohol (and optionally a salt); and

(iv) carrying out a first elution of nucleic acid from the filter, wherein elution comprises incubating the filter in an elution buffer at a temperature from 70 to 90°C followed by centrifugation of the filter; and

(v) carrying out a second elution of nucleic acid from the filter, wherein elution comprises incubating the filter in an elution buffer at a temperature not exceeding 90°C (e.g. 10-90 °C), optionally followed by centrifugation of the filter.

For the elution steps, it will be appreciated that following centrifugation, the eluted buffer (containing the nucleic acid), may be collected and stored/used for further analysis as necessary.

In step (i), the alcohol and salt may be contacted with nucleic acid simultaneously or sequentially. For instance, the salt may be added in the form of a salt solution followed by addition of the alcohol, or vice versa. Alternatively, the salt and alcohol may be provided as components within the same composition (e.g. solution) and the composition either added to the nucleic acid or vice versa.

The salt may be as defined above in respect of the third or fourth aspects of the invention. The salt may for instance comprise a chaotropic agent, as defined herein in respect of the third or fourth aspects of the invention.

The alcohol in step (i) and (iii) may be a corresponding alcohol as described above in relation to the third or fourth aspects of the invention.

In the above method, the method preferably (but need not necessarily) comprises a wash step (iii). That is, the method comprises steps (i), (ii), (iv) and (v) and preferably also comprises the wash step (iii). Skilled artisans will appreciate that the wash step (iii) will assist in providing purer nucleic acid material compared to if the wash step were omitted. It is thus desirable to include wash step (iii). The wash solution will typically contain an alcohol, which may be an alcohol as described above in relation to the third or fourth aspects of the invention.

The wash solution may further comprise a salt. The salt may be as defined in relation to the third or fourth aspect. For instance, the salt is preferably a lithium or guanidinium halide, for example lithium chloride or guanidinium chloride. Treatment with the wash solution is intended to remove impurities, to leave substantially pure nucleic acid immobilised on the filter. The filter is then separated from the liquid, optionally by centrifugation. The salt may be present in the wash solution at a concentration of 0.05 to less than 10 moles per litre, preferably 0.05 to less than 1 moles per litre, preferably 0.1 to 0.9 moles per litre, more preferably 0.6 to 0.9 moles per litre, even more preferably 0.75 to 0.85 moles per litre, and most preferably about 0.8 moles per litre. In some embodiments the wash solution comprises the salt at a concentration of 0.2 to 0.9M.

The filter may be treated in step (iii) with a plurality of wash solutions as described in more detail above in relation to the third and fourth aspects of the invention. The wash solutions may be defined according to any of the third or fourth aspects.

The purification method of the fifth aspect of the present invention advantageously combines the extraction and purification of nucleic acid from a sample. This removes the need for a separate purification step as is commonly known in the art and so reduces the time required to prepare substantially pure nucleic acid.

Ease of use is also improved as fewer manual handling steps are required. A reduction in manual handling steps may also be advantageous in reducing the likelihood of long nucleic acid being broken.

The incubation temperature of step (v) is preferably conducted between 10-90 °C and in embodiments is lower than the incubation temperature of step (iv). In some embodiments the incubation temperature of step (v) is between 10 and 65°C, optionally 20 and 65°C, optionally room temperature. In some embodiments the temperature in step (iv) is from 75 to 85°C, optionally 80°C. It will be appreciated that incubation temperatures exceeding 90 °C could lead to significant denaturing of the nucleic acids.

In the context of the field, the term purification is generally used to describe the removal of impurities from a nucleic acid sample, for example in the purification of nucleic acid from solution or gel slices. Advantageously, the present inventor has found that the method of the above aspect can be used to "clean-up" nucleic acid by preferentially depleting small fragments of nucleic acid from the filter device. In this way a greater proportion of long nucleic acid fragments are retained on the filter device, these long nucleic acid fragments subsequently being eluted. Without wishing to be bound by theory, the present inventors have discovered an improvement in the temperature used in the elution step and believe that the temperature of the first elution step assists in depleting the small fragments.

In embodiments of the methods of the present invention described herein, the number of nucleic acid fragments in the sample obtained following the elution step having a length of from 10kb-165.5kb divided by (i.e. as a ratio) the number of nucleic acid fragments in the sample having a length of from 1.3-10kb is at least 1.5, e.g. at least 1.6 and / or up to 5, e.g. from 1.6 to 3.2.

According to another aspect of the invention, there is provided a kit for isolating nucleic acid from a sample, the kit comprising:

(i) a filter according to the first aspect;
(ii) a salt (preferably a chaoptropic agent, for example a guanidinium salt),
(iii) a wash agent, optionally comprising a lithium salt;

(iii) optionally, an elution buffer; and
optionally a lysing agent.

The elution buffer may comprise (consists essentially of or consists of) a buffered solution of EDTA or a salt thereof. A particular suitable elution solution comprises Tris-HCl in a concentration of about 10mM, and EDTA disodium salt dihydrate at a concentration of about 0.5mM, the solution having a pH of 8 to 9. Another suitable elution solution comprises DNase and RNase-free water.

The chaotropic agent may comprise guanidinium chloride, also known as guanidinium hydrochloride or guanidine hydrochloride.

The lithium salt may be any lithium salt such as described elsewhere herein. The salt is preferably lithium chloride.

The filter may be provided as a component of a nucleic acid extraction column, as defined herein. The agents may be as described in relation to any of the other aspects as defined herein.

The following numbered embodiments are provided:

Embodiment 1. A filter for isolating nucleic acid from a sample, the filter comprising at least a first porous region and a second porous region, the first porous region being arranged to be contacted in use by the sample before the second porous region, wherein the first porous region has a nominal pore size which is greater than the second porous region.

Embodiment 2. The filter according to embodiment 1, wherein the nominal pore size of the second porous region is from $0.5\mu m$ to $1.8\mu m$.

Embodiment 3. The filter according to embodiment 1 or embodiment 2, wherein the nominal pore size of the second porous region is from $0.5\mu m$ to $1.3\mu m$.

Embodiment 4. The filter according to any one of embodiments 1 to 3, wherein the nominal pore size of the first porous region is from $2\mu m$ to $3\mu m$.

Embodiment 5. The filter according to any one of the preceding embodiments, wherein the filter device comprises a plurality of first porous regions.

Embodiment 6. The filter according to any one of the preceding embodiments, wherein the filter further comprises a third porous region, the third porous region being arranged to be contacted in use by the sample after the second porous region, the third porous region having a nominal pore size which is greater than the second porous region.

Embodiment 7. The filter according to embodiment 6, wherein the nominal pore size of the third porous region is substantially equal to the nominal pore size of the first porous region.

Embodiment 8. The filter according to any one of the preceding embodiments comprising a silicate, optionally wherein at least one of the first, second and optionally third porous regions comprises a silicate, optionally further wherein the filter is composed of a silicate.

Embodiment 9. The filter according to embodiment 8, wherein the silicate comprises a silicate glass.

Embodiment 10. The filter according to embodiment 9, wherein the silicate glass comprises, or is, borosilicate glass.

Embodiment 11. The filter according to any one of the preceding embodiments, wherein the first porous region and the second porous region are formed of a fibrous material.

Embodiment 12. The filter according to embodiment 11, wherein the first porous region and the second porous region are each formed of a different fibrous material.

Embodiment 13. The filter according to any preceding embodiment, wherein a thickness of the filter as measured in a direction of sample flow through the filter is from $1000\mu m$ to $2000\mu m$.

Embodiment 14. The filter according to any preceding embodiment, wherein a thickness of the filter as measured in a direction of sample flow through the filter is from $1500\mu m$ to $2000\mu m$.

Embodiment 15. A nucleic acid extraction column comprising the filter according to any one of embodiments 1-14 for isolating nucleic acid from a sample.

Embodiment 16. A method of isolating nucleic acid from a sample using a filter according to any previous embodiment, optionally wherein the method comprises:

(i) contacting a sample comprising nucleic acid with an alcohol and a salt;

(ii) contacting a filter according to any one of embodiments 1 to 14 with the composition comprising nucleic acid obtained following step (i) to provide a filter having nucleic acid bonded thereto;

iii) preferably, treating the filter having the nucleic acid bonded thereto with a wash solution comprising alcohol; and

(iv) eluting at least a portion of the nucleic acid from the filter.

Embodiment 17. The method according to embodiment 16, wherein step (iv) comprises incubating the filter in an elution buffer at a temperature of 10 to 100°C, preferably not exceeding 90 °C.

Embodiment 18. The method according to embodiment 17, wherein the temperature is from 70 to 90°C.

Embodiment 19. The method according to embodiment 17 or embodiment 18, wherein incubation in step (iv) is for a time period of from 30 seconds to 2 minutes.

Embodiment 20. The method according to any one of embodiments 16 to 19 wherein the nucleic acid is eluted from the filter in step (iv) by centrifugation.

Embodiment 21. The method according to embodiment 20, wherein the g-force of centrifugation is from 600 to 800g.

Embodiment 22. The method according to any one of embodiments 16 to 21, wherein the salt comprises a chaotropic agent, optionally a guanidinium salt.

Embodiment 23. The method according to any one of embodiments 16 to 22 wherein the filter is provided as a component of a nucleic acid extraction column.

Embodiment 24. A method of isolating nucleic acid from a sample, the method comprising:

(i) contacting a sample comprising nucleic acid with an alcohol and a salt; and

(ii) contacting a filter with the composition comprising nucleic acid obtained following step (i) to provide a filter having nucleic acid bonded thereto; and

(iii) preferably, treating the filter having the nucleic acid bonded thereto with a wash solution comprising alcohol; and

(iv) eluting at least a portion of the nucleic acid from the filter, wherein elution comprises incubating the filter in an elution buffer at a temperature of from 70 to 90°C.

Embodiment 25. The method according to embodiment 24, wherein incubation in step (iv) is performed at a temperature of from 75 to 85°C.

Embodiment 26. The method according to embodiment 24 or 25, wherein incubation in step (iv) is for a time period of from 30 seconds to 2 minutes.

Embodiment 27. The method according to any one of embodiments 24 to 26 wherein eluting the nucleic acid from the filter in step (iv) comprises centrifugation.

Embodiment 28. The method according to embodiment 27, wherein g-force of centrifugation is from 600 to 800g.

Embodiment 29. The method according to any one of embodiments 24 to 28, further comprising: (v) carrying out a second elution of nucleic acid from the filter, wherein elution comprises incubating the filter in an elution buffer at a temperature not exceeding 90°C, optionally followed by centrifugation of the filter.

Embodiment 30. A method of purifying nucleic acid isolated from a sample, the method comprising:

(i) contacting the nucleic acid with an alcohol and a salt;

(ii) contacting a filter with the composition comprising nucleic acid obtained following step (i) to provide a filter having nucleic acid bonded thereto;

(iii) preferably, treating the filter having the nucleic acid bonded thereto with a wash solution comprising alcohol; and

(iv) carrying out a first elution of nucleic acid from the filter, wherein elution comprises incubating the filter in an elution buffer at a temperature of from 70 to 90°C, optionally followed by centrifugation of the filter; and

(v) carrying out a second elution of nucleic acid from the filter, wherein elution comprises incubating the filter in an elution buffer at a temperature not exceeding 90°C, optionally followed by centrifugation of the filter.

Embodiment 31. The method according to embodiment 30, wherein the incubation temperature of step (v) is lower than the incubation temperature of step (iv).

Embodiment 32. The method according to embodiment 31, wherein the incubation temperature of step (v) is from 20 to 65°C.

Embodiment 33. The method according to any one of embodiments 24 to 32, wherein the salt in step (i) comprises a chaotropic agent, optionally a guanidinium salt.

Embodiment 34. The method according to any one of embodiments 16 to 33, wherein the salt in step (i) is provided in an amount such that the composition comprising nucleic acid formed from step (i) contains a concentration of from 0.05 to 10M, optionally 0.1 to 3M, preferably 0.1 to 2M, more preferably 0.5 to 0.9M, even more preferably 0.6 to 0.7M of the salt.

Embodiment 35. The method according to any one of embodiments 16 to 34, wherein the alcohol in step (i) comprises a $C_{1-3}$ alkanol, preferably isopropanol or ethanol.

Embodiment 36. The method according to embodiment 35 wherein the alcohol is provided in an amount such that the composition comprising nucleic acid formed from step (i) contains from 25 to 40% by volume, optionally from 25 to 30% by volume of the alcohol.

Embodiment 37. The method according to any one of embodiments 24 to 36 wherein the filter is a filter according to any one of embodiments 1 to 14.

Embodiment 38. The method according to any one of embodiments 24 to 37 wherein the filter is provided as a component of a nucleic acid extraction column.

Embodiment 39. A kit for isolating nucleic acid from a sample, the kit comprising:

(i) a filter according to any one of embodiments 1 to 14;
(ii) a salt (preferably a chaotropic agent, for example a guanidinium salt),
(iii) a wash agent, optionally comprising lithium;
(iii) optionally, an elution buffer; and
optionally a lysing agent.

**Description of the figures**

The invention will now be illustrated with reference to the following non-limiting Examples 1-6 and Figs. 1-8 of the accompanying drawings, which show:

Figure 1 shows a comparison of the ratio of longer nucleic acid to shorter nucleic acid isolated using filters of the invention;

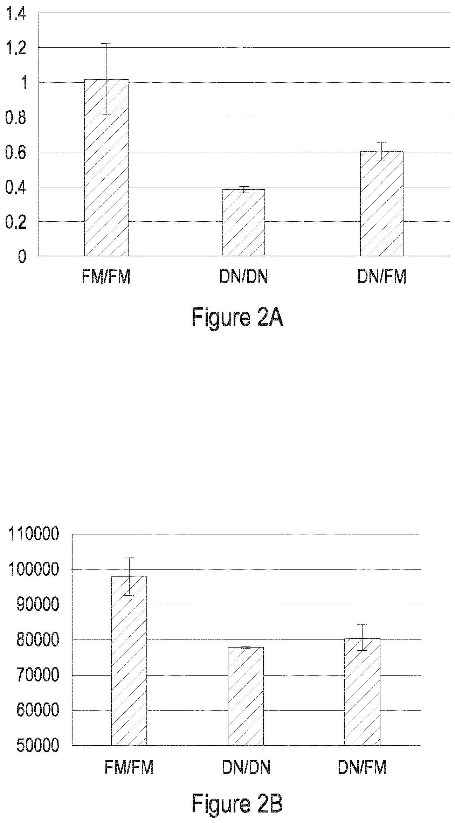

Figure 2 (A) shows a comparison of the ratio of long to short DNA obtained using the filter of the present invention and/or the method of the present invention versus the DNeasy filter and method at an incubation temperature of room temperature, (B) shows a comparison of the average base pair length of DNA obtained using the filter of the present invention and/or the method of the present invention versus the DNeasy filter and method at an incubation temperature of room temperature, (C) shows a comparison of the ratio of long to short DNA obtained using the filter of the present invention and/or the method of the present invention versus the DNeasy filter and method at an incubation temperature of 80°C and (D) shows a comparison of the average base pair length of DNA obtained using the filter of the present invention and/or the method of the present invention versus the DNeasy filter and method at an incubation temperature of 80°C;

Figure 3 shows the effect of the concentration of LiCl on the ratio of large to small DNA obtained using a method according to the invention;

Figure 4 shows the effect of different alcohols on the ratio of large to small DNA obtained using a method according to the invention;

Figure 5 shows the effect of the temperature of the second of two elution steps upon the ratio of long to short DNA fragments obtained using a method and filter according to the present invention;

Figure 6 shows the effect of the temperature of the second of two elution steps upon the ratio of long to short DNA fragments obtained using a method according to the present invention on different filters. 65°C and 80°C were tested against each filter. (A) shows the ratio for the 4F filter, (B) shows the ratio for the 2F filter, (C) shows the ratio for the DF filter, (D) shows the ratio for the 2DF filter, (E) shows the ratio for the F2D filter and (F) shows the ratio for the DFD filter;

Figure 7 shows the effect of two elution steps having incubation temperatures of 80°C upon the ratio of long to short DNA obtained using different filters; and

Figure 8 shows the ability of filters of the invention to purify longer DNA relative to known size exclusion protocols.

**Experimental methods and performance analysis**

**Example 1**

**Ratio of DNA extracted**

The inventor decided to study the impact of filters against the invention on the ratio of long to short DNA obtained in a DNA isolation protocol. The effect of the filters of the invention was compared to filters not according to the first aspect.
The example was otherwise carried out using a method in accordance with the third aspect of the invention. Briefly, white blood cells were isolated from a 1ml sample of horse blood and the following procedure was followed:

(a) Ice-cold hypertonic solution added to sample and the sample incubated at room temperature for 5 to 10 minutes to destroy the red blood cells;
(b) Sample is centrifuged at a speed of 250 $\times$ G for 3 minutes; supernatant (containing unwanted red blood cell material) is discarded without disturbing the pellet (containing white blood cells);
(c) Sample (pellet containing white blood cell material) is contacted with a lysis solution comprising 0.8M LiCl, 0.5% SDS, proteinase K and 10mM EDTA and pipette to mix to lyse the sample;
(d) Sample contacted with a 2M solution of the chaotropic agent GuHCl and vortex.
(e) 75% isopropanol added and vortex;
(f) Transfer the sample to a nucleic acid extraction column, in this embodiment a spin column comprising a filter;
(g) Spin at 4,722 $\times$ g for 1 min
(h) Discard the flow-through
(i) Add wash solution comprising LiCl and 50% ethanol to the spin column and centrifuge at 4722g for 1 min. Discard the flow-through.

(j) Add 90% ethanol to the spin column and centrifuge at 14,462 × g for 3 mins. Discard the flow-through.

(k) Centrifuge the spin column at 14,462 × g for 1 min. Discard the flow-through.

(l) Add elution buffer (10mM Tris HCL, 0.5mM EDTA) to the spin column and incubate at room temperature for 1 min. Elute at 1,180g for 2 mins to obtain fraction A; and

(m) Optionally repeat (l), to obtain fraction B.

Steps (a) and (b) are optional steps used to remove red blood cells from a whole blood sample.

It essentially acts to pellet the white blood cells from the sample. The white blood cells are then be lysed in step (c). As the skilled person will appreciate, step (c) is also optional if lysis of the sample is not required.

Porous regions suitable for use in the present invention are detailed in Table 1. All of the porous regions in Table 1 are available from GE Life Sciences (USA). Other suitable porous regions, for example, in the form of filter sheets, will be commercially available to the skilled person.

### Table 1

| Grade | A | B | D | F |
|---|---|---|---|---|
| Nominal pore size (based on a 98% efficiency in $\mu$m) | 1.6 | 1.0 | 2.7 | 0.7 |

Each of the filters can be cut to size as necessary and is formed of borosilicate glass.

To form each filter, a variety of different porous region combinations were tested (see Table 1 for information on each porous region and Table 2 for information on the filters tested). Briefly, each of the porous regions was cut to a 0.38cm$^2$ diameter for insertion into the spin column. Since the porous regions were in a membrane form, to form each filter the porous regions were combined together using an O-ring formed of Purell HP570R (Lyondellbasell, Milton Keynes, UK) to secure the filters together at their peripheries.

The nomenclature used indicates the positioning of each porous region relative to contact by the sample in use. Thus, for the DF filter, the D porous region is the "top" porous region, i.e. the porous region closest to the open end of the spin column and so the porous region which will be contacted first by the sample in use, while the F porous region is underneath the D porous region. For the F2D filter, the F porous region is the "top" porous region, with the 2 D porous regions underneath.

For the present experiments, F porous regions were obtained from Porex Corporation, Aachen, Germany), while D porous regions were obtained from Whatman (GE Life Sciences (USA)). However, the skilled person will appreciate that equivalent porous regions are available from a number of other commercial suppliers.

### Table 2

| Filter of the invention | Content of filter of the invention | Comparative filter | Content of comparative filter |
|---|---|---|---|
| DF | 1 × D and 1 × F porous region | 4F | 4 × F porous regions |
| 2DF | 2 × D porous region and 1 × F porous region | 2F | 2 × F porous regions |
| DFD | 1 × D porous region, 1 × F porous region and 1 × D porous region | F2D | 1 × F porous region and 2 × D porous regions |

Figure 1 shows the effect of the filters of Table 2 on the ratio of long to short DNA fragments obtained using the above protocol.

The number of DNA molecules of a particular size extracted in the elution step (l) was analysed using the FEMTO Pulse parallel capillary electrophoresis instrument (Advanced Analytical Technologies, Inc., Milton Keynes, UK). This instrument calculates nanomoles/Lt for different groups of DNA smear.

The analysis calculated the number of DNA fragments for the smear brackets: 1.3kb to 10kb, and 10kb to 165.5kb. The 10kb to 165.5kb bracket was then divided by the 1.3kb to 10kb bracket to produce the ratios shown in Figure 1. Hence, the ratio will be understood to relate to the "10kb cut-off". Average values are shown with standard deviation from duplicate samples.

These ratios give an indication of the relative abundance of molecules of specific sizes. The 1.3 and 165.5kb sizes were chosen because these are the lower and higher marker of the FemtoPulse ladder. The cut-off was chosen to be 10kb, because molecules up to that size can severely overwhelm long read sequencing read-outs. The group of 10kb to 165.5kb was chosen because it covers the usual range for long-read sequencing (25 to 35kb), as externally validated together with fragments of 50 to 165.5kb which are considered to be very long.

As the skilled person will appreciate, a ratio of more than 1 indicates a higher number of longer-length DNA fragments compared to shorter-length DNA fragments. A ratio of less than 1 indicates a greater number of shorter-length DNA fragments compared to longer-length DNA fragments.

Figure 1A shows the ratios obtained for the comparative filters 4F and 2F as compared to a filter of the invention DF, i.e. having a porous region with a greater nominal pore size above a porous region having a smaller nominal pore size. This Figure clearly shows that using the DF filter resulted in a greater ratio (1.91 as compared to 1.10 for 4F and 0.76 for 2F) of longer to shorter DNA fragments.

Figure 1B shows the ratios obtained for the comparative filter FD compared to a filter of the invention, DF. As for Figure 1A, the ratio of longer to shorter DNA fragments obtained using the DF (1.96) filter was considerably greater than the ratio obtained for the FD filter (1.10).

Figure 1C shows the ratios obtained for the comparative filters 2F and F2D as compared to filters of the invention 2DF and DFD. Both 2DF (2.84) and DFD (3.03) gave considerably greater ratios than the comparative filters (1.17 for 2F and 0.91 for F2D), with DFD obtaining the greatest ratio.

These data demonstrate that filters of the present invention provide an improved ratio of longer to shorter nucleic acid fragments when used in nucleic acid isolation protocols. All data is derived from the first of two elutions after pre-incubation for 1 minute at 65°C (rather than room temperature as specified in step I).

**Example 2**

**Extracted DNA ratio and length comparison to DNeasy filter**

The filter of the invention, in this embodiment the DFD filter as described in Example 1 and the method as described in Example 1 were then compared to known filters and methods of the DNeasy DNA blood and tissue extraction protocol and kit (Qiagen, Germantown, MD, US). As for Example 1, samples were analysed using a FEMTO Pulse machine.

The samples were white blood cells isolated from 1ml of horse blood.

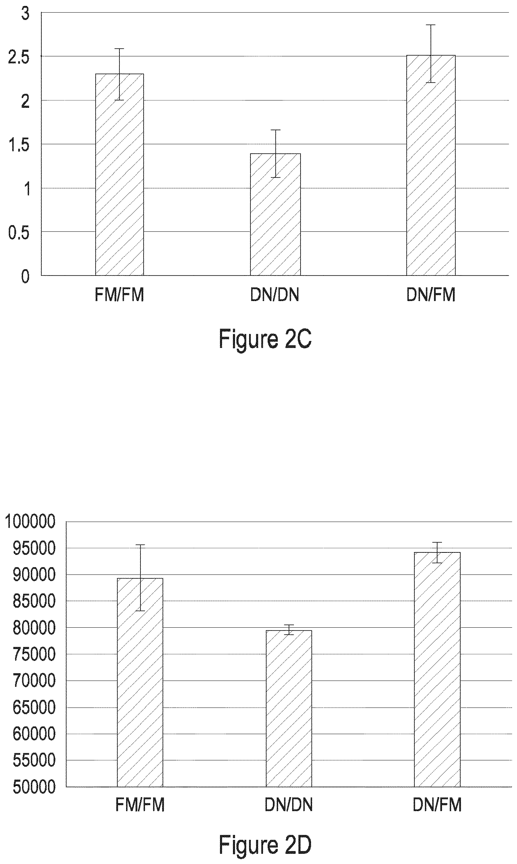

The inventor wished to determine if the filter of the invention provided improved isolation of longer DNA regardless of the protocol used with the filter. The results are shown in Figure 2. These results show the ratio (Figures 2A and C) of long to short DNA isolated and the average base pair length of isolated DNA fragments (2B and 2D) in the eluate. For this analysis the "cut off" for the ratio was 20KB; i.e. the 20kb to 165.5kb bracket was then divided by the 1.3kb to 20kb bracket to produce the ratios shown. The average base pair length was calculated for the isolated DNA population in the 20 to 165.5kb bracket.

For all protocols, the elution step was performed using a pre-elution incubation step of either room temperature (Figures 2A and 2B) or 80°C (Figures 2C and 2D). The results from the second elution are presented.

The x labels define the following conditions:

FM/FM = Method according to Example 1/ DFD filter
DN/DN = DNeasy protocol and reagents from kit/Dneasy filter from kit
DN/FM = DNeasy protocol and reagents from kit/DFD filter

The results show that regardless of the incubation temperature or protocol and reagents used, the DFD filter provided an improved ratio of isolated long to short DNA fragments and an increased average base pair length compared to the DNeasy filter. This shows that the filter of the present invention provides improved results with various methods of isolation. The ratio and average base pair length were further improved when the DFD filter was used with the method of the present invention as exemplified in Example 1.

For all filters and methods, the ratio obtained and the average base pair length were improved by using a pre-elution incubation temperature of 80°C as compared to room temperature.

**Example 3**

**Conditions**

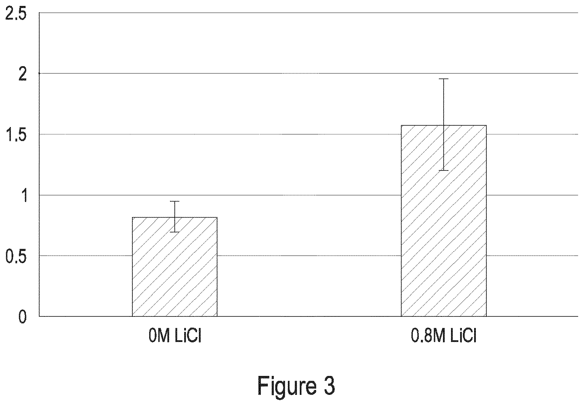

The effect of different buffers in the present protocol was then considered. Figure 3 shows the effect of Lithium Chloride provided on the ratio of DNA isolated. For this figure, a DNA extraction protocol was carried out in accordance with the protocol of Example 1 on a 1ml sample of horse blood, except that a concentration of either 0M LiCI or 0.8M LiCI was used in the solution of step (c) and that incubation in step (I) was carried out at 65°C. For this protocol, a 4F filter (i.e. a comparative filter) was used. In this way the effect of the method alone could be assessed.

Ratios were determined using the same cut-offs as for Figure 1.

Figure 3 demonstrates that the inclusion of LiCl in a solution of a method of the invention increases the ratio of long to short DNA fragments obtained. This demonstrates that the method of the invention is able to provide an improved ratio of long to short DNA fragments independently of the filter of the present invention. All data is derived from duplicate samples (white blood cells from 1ml of horse blood) and shows results from a first elution after pre-incubation for 1 minute at 65°C at a cut-off of 10kb.

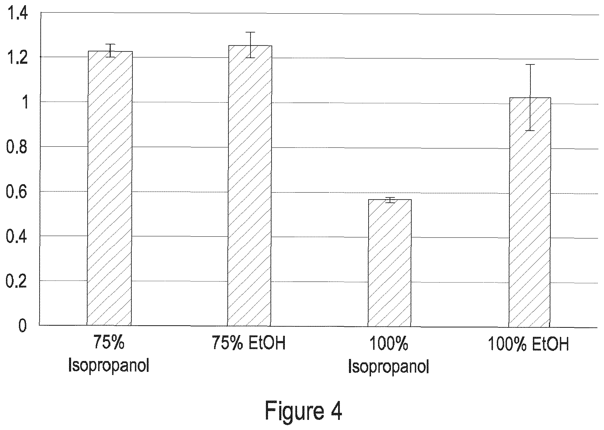

Figure 4 shows the effect of different alcohols and alcohol percentages upon the ratio of DNA isolated. As for Figure 3, for this Figure a DNA extraction protocol was carried out in accordance with the protocol of Example 1 on a 1ml sample of horse blood, except that the incubation step (I) was carried out at 65°C. However, 75% isopropanol, 75% ethanol, 100% isopropanol and 100% ethanol were tested in step (e) of the protocol. Ratios were determined using the same cut-offs as for Figure 1. Where the alcohol provided was 100% isopropanol or 100% ethanol, the resulting concentration of alcohol in the composition that was submitted to filtration was 37% v/v. Where the alcohol was used was 75% isopropanol or 75% ethanol, the resulting concentration of alcohol in the nucleic acid composition that was submitted to filtration was about 28.57% by volume.

The use of 75% ethanol provided the greatest ratio (1.26), followed by 75% isopropanol (1.23), 100% ethanol (1.03) and 100% isopropanol (0.57). This also demonstrates that parameters of the method of the invention affect the ratio obtained independently of the filter of the invention. All data is derived from duplicate samples (white blood cells from 1ml of horse blood) and shows results from a first elution after pre-incubation for 1 minute at 65°C at a cut-off of 10kb.

## Example 4

### Temperature

The effect of the incubation temperature in the elution step of the method of isolating DNA was then considered.

The protocol detailed in Example 1 was used on a sample of $3\mu g$ of DNA of a known mixture of long and short fragments. In this way the efficiency of the improved retention of the longer fragments could be directly compared to the ratio of DNA in the input sample. The filter used was the DFD filter.

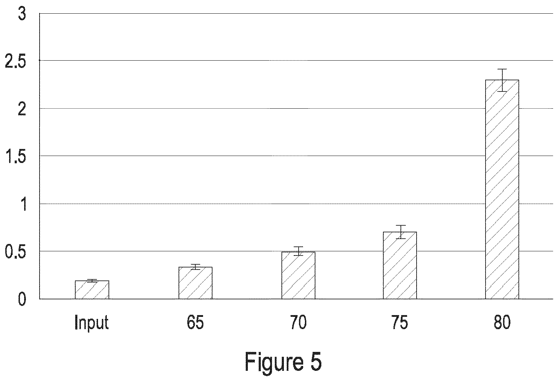

In this example, two elution steps were carried out; each elution step comprised a pre-elution incubation at the temperatures indicated in Figure 5 for 1 minute. The number of DNA molecules in the second eluate was then analysed using the FEMTO Pulse parallel capillary electrophoresis instrument. A 20KB cut-off was used to calculate the ratio as described in Example 2.

Figure 5 shows the ratios obtained. While the method according to the present invention improved the ratio of long to short DNA as compared to the input at all pre-elution temperatures, the increase in long to short DNA ratio was most considerable using an 80°C pre-elution temperature.

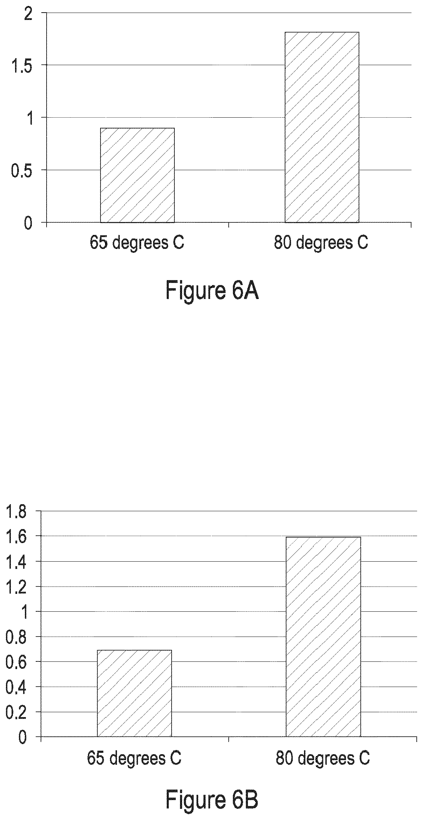

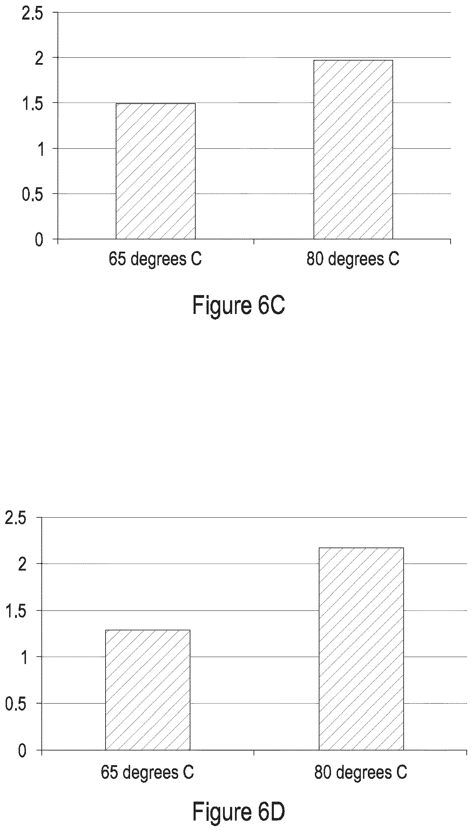

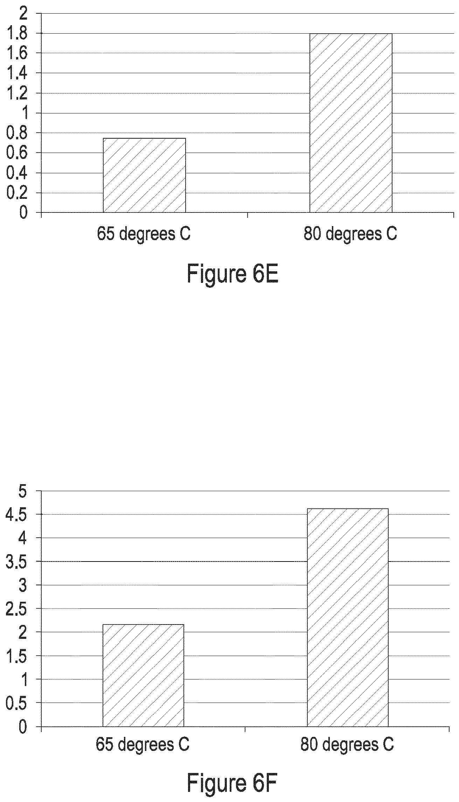

The effect of the pre-elution temperature of 80°C was then tested on filters of the invention and comparative filters. The results are shown in Figure 6. For these data, the method according to Example 1 was carried out on white blood cells from 1ml samples of horse blood. However, two elution steps were carried out, each with a pre-elution incubation at either 65 or 80°C for 1 minute. The DNA content of the second eluate was then analysed using the FEMTO Pulse parallel capillary electrophoresis instrument. A 20kb cut-off was used.

A) shows the ratio for the 4F filter, (B) shows the ratio for the 2F filter, (C) shows the ratio for the DF filter, (D) shows the ratio for the 2DF filter, (E) shows the ratio for the F2D filter and (F) shows the ratio for the DFD filter. For all filters, the 80°C incubation step greatly increased the ratio of longer DNA fragments obtained, indicating that the method of the invention using an increased temperature for incubation prior to elution gives improved results regardless of the filter used. This effect is especially pronounced for the DFD filter (F).

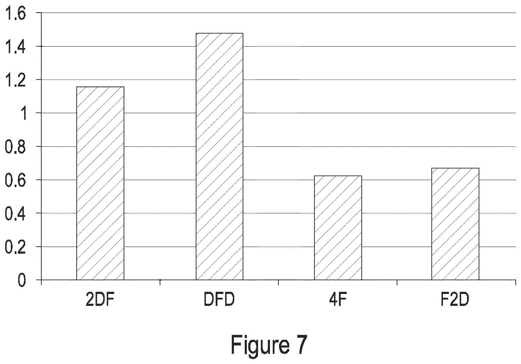

Figure 7 shows the ratio of long to short DNA obtained from a second eluate using different filters following two pre-eluate incubations at 80°C. For this data, a 40kb cut-off was used, i.e. the ratio was calculated by dividing the 40kb to 165.5kb bracket by the 1.3kb to 40kb bracket. By increasing the cut-off ratio, this assists in showing which filters are of most utility in isolating the particularly long DNA fragments. The method of Example 1 was otherwise used.

Figure 7 shows that the filters of the invention, DFD (1.48) and 2DF (1.16), gave increased ratios compared to the comparative filters 4F (0.62) and F2D (0.67). This demonstrates that the filters of the invention give improved ratios to the comparative filters at a temperature of 80°C as well as a lower temperature of 65°C as indicated in Figure 1.

## Example 5

### Utility of the method of the present invention in purifying nucleic acid

In addition to improving the proportion of long DNA fragments obtained, it is useful to be able to deplete DNA fragments under a certain cut off, for example 10kb. This further improves the purity and/or utility of DNA samples, such as library samples or DNA extracts from known nucleic acid isolation technologies that are less suitable for the optimal extraction of longer nucleic acid for long-read sequencing technologies such as ONTs and PacBio.

The capability of the present protocol and filters to exclude short DNA fragments as compared to a known size-selection SPRI (solid phase reversible immobilisation beads) bead protocol (Agencourt AMPure XP beads, Beckman Coultier) was considered. SPRI beads are recommended by ONT for use as a quick size selection process post-extraction of DNA for depletion of up to 2kb.

The SPRI protocol was as follows: 3μg of DNA in 50μl is mixed with 35μl of beads (in 10mM Tris-HCl, 1mM EDTA, 1.6M NaCl, 11% PEG 8000) and the solution is reversed top to bottom at room temperature for 10mins. It is then spun briefly for a few seconds and pelleted on a magnet. The supernatant is pipetted and the pellet is washed with 200μl of 70% EtOH. After the EtOH is removed the EtOH wash is repeated and the pellet is allowed to dry to remove any residual EtOH. The pellet is re-suspended in 50μl of TE buffer (10mM Tris-HCl, pH 8, 1 mM EDTA) and incubated for 1min at 50°C and 5mins at RT. The beads are then pelleted on a magnet and the supernatant is used downstream.

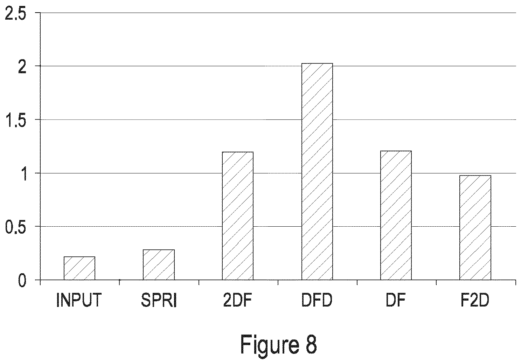

The ability of various filters to purify DNA was compared to the 0.7x SPRI size selection system. 3μg of a mixture of low and high molecular weight DNA was used as an input. Briefly 3μg of DNA in a volume of up to 100μl of elution buffer was mixed with a premixed solution of 150μl solution comprising 0.8M LiCl, 0.5% SDS, proteinase K and 10mM EDTA, 175μl of a 2M solution of the chaotropic agent GuHCl BS and 200μl 75% Isopropanol which was immediately loaded onto the column following the standard spin protocol. Prior to each elution (the protocol having two elutions) the elution buffer was incubated for 1 minute at 80°C. The second eluate was analysed using a 20kb cut-off. As Figure 8 shows, all filters performed better than the SPRI with the DFD filter providing the greatest ratio of 2.03. Elevated pre-elution incubation temperatures may contribute to the improved size selection and hence purification by facilitating detachment of the smaller molecules from the matrix, therefore they may be more readily depleted.

Accordingly, the methods of the present invention may be used to isolate and/or to purify nucleic acid. When used to purify a nucleic acid sample, it may be beneficial to use the second elution. This is because, without wishing to be bound by theory, the present inventor believes that the first elution is likely to contain the smaller nucleic acid fragments which are less suitable for downstream sequencing applications.

### Example 6

### Sequencing analysis

A protocol in accordance with Example 1 was carried out using a DFD filter device in a spin column to isolate DNA (using eluate B).

An LSK109 library kit (Oxford Nanopore Technologies, Oxford, UK) was then used to prepare a sequencing library from each set of DNA. The LSK109 method results in the repair of DNA ends and dA-tailing. Sequencing adapters, supplied in the LSK109 kit, are then ligated onto the prepared ends. After this a MinION flow cell (Oxford Nanopore Technologies) was loaded with the DNA library to sequence the library.

According to protocol instructions, sequencing with the MinION flow cell can be run for six hours. However, as the flow cell has a run life of approximately 48 hours, the flow cell can be left running for this length of time. This provides more nucleic acid sequences for bioinformatics analysis.

Thus, sequencing using the MinION flow cell was run for 48hrs and the sequencing summaries for the first 6 and 48hrs were analyzed by Nanoplot software (https://pypi.org/proiect/NanoPlot/) which provided the values. Q score stands for a measure of individual base call accuracy, and is usually around 10 for ONT sequencing. ONT recommends using a cut-off of 7 for analysis:
After 6hrs of sequencing the statistics were; average length: 27,080bp and N50: 47,606bp and after 48hrs of sequencing the average length: 30,096bp and the N50: 49,590bp.

As the skilled person will appreciate, N50 defines the assembly quality in terms of contiguity. The N50 is defined as the sequence length of the shortest contig at 50% of the total genome length.

Oxford Nanopore Technologies generally recommends using an SPRI bead step between DNA extraction and preparation of the library to remove DNA strands below 2kb which would otherwise overwhelm the Minion flow cell. However, this step was not required for the present method using the 2DF or DFD filter due to the ability of the 2DF or DFD filter to clean-up and size-select the DNA during extraction.

Further sequencing analysis was carried out on white blood cell samples pelleted from 2ml of horse blood. For these experiments, DNA was extracted in parallel using a protocol in accordance with Example 1 (using eluate B). Either a 2DF or a DFD filter device was used.

An LSK109 library kit was used to prepare a sequencing library (no post-extraction SPRI step was included) followed by sequencing either 20μl of DNA in a final volume of 60μl (in accordance with the manufacturer's instructions), or 47μl of DNA in a final volume of 60μl using a Minion flow cell for either 6 or 48 hours.

The results are shown in Table 3 below.

**Table 3**

| | 20microLt input | | 47microLt input | |
|---|---|---|---|---|
| | **2DF column, 6hrs sequencing** | **DFD column, 6hrs sequencing** | **DFD column, 6hrs sequencing** | **DFD column, 48hrs sequencing** |
| **Mean read length (bp)** | **16556.3** | **15423.7** | **27080.8** | **30096.3** |
| **Mean read quality** | 10.5 | 10.3 | 10.2 | 10.1 |
| **Median read length (bp)** | **5475** | **5195** | **18916** | **23076** |
| **Median read quality** | 10.7 | 10.6 | 10.4 | 10.4 |
| **Number of reads** | 68894 | 100263 | 54561 | 156879 |
| **N50 (bp)** | **39776** | **42388** | **47606** | **49590** |
| **Total bases** | 1140627470 | 1546425755 | 1477553802 | 4721479216 |
| **Top 5 reads (bp) with quality score** | 182110 (11.6) | 234475 (11.7) | 235546 (9.8) | 308648 (10.8) |
| | 181953 (10.2) | 228843 (10.1) | 223082 (9.5) | 246632 (9.9) |
| | 174586 (9) | 216574 (11) | 217688 (9.8) | 235546 (9.8) |
| | 168110 (8) | 212367 (9.2) | 211212 (11.7) | 233787 (8.7) |
| | 165681 (8.9) | 208977 (11.1) | 208171 (9.4) | 231600 (9.1) |

DNA obtained using the DFD filter had a greater N50 than the 2DF filter, of ~42kb vs 2DF's ~40kb when the 20µl input was analysed. For the 47µl input, the N50 goes up to ~48 and ~50kb for the 6 and 48hrs runs.

These results indicate that use of the method of the present invention to isolate and purify nucleic acid can lead to improved results downstream, for example for sequencing applications. The inventor believes this is due to the increased ratio of long to short DNA fragments in the sample. In addition, the inventor has found that because of the improved ratio, there is less interference from smaller DNA fragments and so an increased volume of DNA can be used as input for the sequencing. This, in turn, provides improved sequencing results.

**References**

Cavalier, et al. 2015 - Cavelier L, Ameur A, Haggqvist S, Höijer I, Cahill N, Olsson-Strömberg U, Hermanson M. (2015) Clonal distribution of BCR-ABL1 mutations and splice isoforms by single-molecule long-read RNA sequencing. BMC Cancer. 15:45. doi: 10.1186/s12885-015-1046-y.

**Claims**

1. A method of isolating nucleic acid from a sample, the method comprising:

(i) contacting a sample comprising nucleic acid with an alcohol and a salt, wherein the salt comprises a chaotropic agent; and
(ii) contacting a filter with the composition comprising nucleic acid obtained following step (i) to provide a filter having nucleic acid bonded thereto; and
(iii) preferably, treating the filter having the nucleic acid bonded thereto with a wash solution comprising alcohol; and

(iv) eluting at least a portion of the nucleic acid from the filter, wherein elution comprises incubating the filter in an elution buffer at a temperature of from 70 to 90°C.

2. The method according to claim 1:

   (A) wherein incubation in step (iv) is performed at a temperature of from 75 to 85°C; and / or
   (B) wherein incubation in step (iv) is for a time period of from 30 seconds to 2 minutes.

3. The method according to any one of claims 1 to 2 wherein eluting the nucleic acid from the filter in step (iv) comprises centrifugation, optionally wherein g-force of centrifugation is from 600 to 800g.

4. The method according to any one of claims 1 to 3, further comprising: (v) carrying out a second elution of nucleic acid from the filter, wherein the second elution comprises incubating the filter in an elution buffer at a temperature not exceeding 90°C, optionally followed by centrifugation of the filter.

5. A method of purifying nucleic acid isolated from a sample, the method comprising:

   (i) contacting the nucleic acid with an alcohol and a salt, wherein the salt comprises a chaotropic agent;
   (ii) contacting a filter with the composition comprising nucleic acid obtained following step (i) to provide a filter having nucleic acid bonded thereto;
   (iii) preferably, treating the filter having the nucleic acid bonded thereto with a wash solution comprising alcohol; and
   (iv) carrying out a first elution of nucleic acid from the filter, wherein elution comprises incubating the filter in an elution buffer at a temperature of from 70 to 90°C, optionally followed by centrifugation of the filter; and
   (v) carrying out a second elution of nucleic acid from the filter, wherein elution comprises incubating the filter in an elution buffer at a temperature not exceeding 90°C, optionally followed by centrifugation of the filter.

6. The method according to claim 5, wherein the incubation temperature of step (v) is lower than the incubation temperature of step (iv), optionally wherein the incubation temperature of step (v) is from 20 to 65°C.

7. The method according to any one of claims 1 to 6:

   (A) wherein the salt in step (i) comprises a guanidinium salt; and / or
   (B) wherein the salt in step (i) is provided in an amount such that the composition comprising nucleic acid formed from step (i) contains a concentration of from 0.05 to 10M, optionally 0.1 to 3M, preferably 0.1 to 2M, more preferably 0.5 to 0.9M, even more preferably 0.6 to 0.7M of the salt.

8. The method according to any one of claims 1 to 7, wherein the alcohol in step (i) comprises a $C_{1-3}$alkanol, preferably isopropanol or ethanol, optionally wherein the alcohol is provided in an amount such that the composition comprising nucleic acid formed from step (i) contains from 25 to 40% by volume, optionally from 25 to 30% by volume of the alcohol.

9. The method according to any one of claims 1 to 8, wherein the filter comprises at least a first porous region and a second porous region, the first porous region being arranged to be contacted in use by the sample before the second porous region, wherein the first porous region has a nominal pore size which is greater than the second porous region.

10. The method according to claim 9:

    (A) wherein the nominal pore size of the second porous region is from $0.5\mu$m to $1.8\mu$m, optionally from $0.5\mu$m to $1.3\mu$m; and / or
    (B) wherein the nominal pore size of the first porous region is from $2\mu$m to $3\mu$m.

11. The method according to claim 9 or claim 10:

    (A) wherein the filter comprises a plurality of first porous regions; and / or
    (B) wherein the filter further comprises a third porous region, the third porous region being arranged to be contacted in use by the sample after the second porous region, the third porous region having a nominal pore size which is greater than the second porous region, and optionally wherein the nominal pore size of the third porous region is substantially equal to the nominal pore size of the first porous region.

12. The method according to any one of claims 9 to 11, wherein the filter comprises a silicate, optionally wherein at least one of the first, second and optionally third porous regions comprises a silicate, optionally further wherein the filter is composed of a silicate.

13. The method according to claim 12, wherein the silicate comprises a silicate glass, optionally wherein the silicate glass comprises, or is, borosilicate glass.

14. The method according to any one of claims 9 to 13, wherein:

(A) the first porous region and the second porous region are formed of a fibrous material, optionally wherein the first porous region and the second porous region are each formed of a different fibrous material; and / or
(B) a thickness of the filter as measured in a direction of sample flow through the filter is from $1000\mu m$ to $2000\mu m$, optionally wherein a thickness of the filter as measured in a direction of sample flow through the filter is from $1500\mu m$ to $2000\mu m$.

15. The method according to any one of claims 1 to 14 wherein the filter is provided as a component of a nucleic acid extraction column.

Figure 1A

Figure 1B

Figure 1C

Figure 2A

Figure 2B

Figure 2C

Figure 2D

Figure 3

Figure 4

Figure 5

Figure 6A

Figure 6B

Figure 6C

Figure 6D

Figure 6E

Figure 6F

Figure 7

Figure 8

Europäisches
Patentamt

European
Patent Office

Office européen
des brevets

## EUROPEAN SEARCH REPORT

Application Number

EP 21 20 5118

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/157650 A1 (WAKO PURE CHEM IND LTD [JP]; WAKO LIFE SCIENCES INC [US]) 15 October 2015 (2015-10-15) * the whole document * | 1-15 | INV. C12N15/10 |
| Y | WO 2015/120447 A1 (ZYMO RES CORP [US]) 13 August 2015 (2015-08-13) * the whole document * | 1-15 | |
| Y | JP 2005 224167 A (GL SCIENCES INC) 25 August 2005 (2005-08-25) * the whole document * | 1-15 | |
| Y | WO 2007/036564 A2 (AJ INNUSCREEN GMBH [DE]; TIMO HILLEBRAND [DE]) 5 April 2007 (2007-04-05) * the whole document * | 1-15 | |
| Y | LIU LIJIA ET AL: "Extraction of genomic DNA using a new amino silica monolithic column", JOURNAL OF SEPARATION SCIENCE, WILEY, DE, vol. 32, no. 15-16, 1 August 2009 (2009-08-01), pages 2752-2758, XP009189793, ISSN: 1615-9306 * the whole document * | 1-15 | TECHNICAL FIELDS SEARCHED (IPC) C12N |
| Y | US 2006/099605 A1 (HALL GERALD E JR [US] ET AL) 11 May 2006 (2006-05-11) * claim 11 * | 1-15 | |
| Y | WO 2017/203225 A1 (REVOLUGEN LTD [GB]) 30 November 2017 (2017-11-30) * example 2 * | 1-15 | |

-/--

The present search report has been drawn up for all claims

| Place of search | Date of completion of the search | Examiner |
|---|---|---|
| Munich | 28 March 2022 | Young, Craig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 985 112 A1**

Europäisches
Patentamt

European
Patent Office

Office européen
des brevets

# EUROPEAN SEARCH REPORT

**Application Number**

**EP 21 20 5118**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2005/026175 A1 (LINK JOHN [US] ET AL) 3 February 2005 (2005-02-03) * paragraph [0077]; claim 5 * | 1-15 | |
| Y | US 2005/026159 A1 (ROBBINS CLAUDIA A [US] ET AL) 3 February 2005 (2005-02-03) * paragraph [0039]; claim 1 * | 1-15 | |
| A | CLOSE ELIZABETH D ET AL: "Nucleic acid separations using superficially porous silica particles", JOURNAL OF CHROMATOGRAPHY A, ELSEVIER, AMSTERDAM, NL, vol. 1440, 23 February 2016 (2016-02-23), pages 135-144, XP029444481, ISSN: 0021-9673, DOI: 10.1016/J.CHROMA.2016.02.057 * the whole document * | 1-15 | |
| Y | VANDEVENTER PETER E. ET AL: "DNA Adsorption to and Elution from Silica Surfaces: Influence of Amino Acid Buffers", JOURNAL OF PHYSICAL CHEMISTRY PART B, vol. 117, no. 37, 19 September 2013 (2013-09-19), pages 10742-10749, XP55906175, US ISSN: 1520-6106, DOI: 10.1021/jp405753m Retrieved from the Internet: URL:https://www.ncbi.nlm.nih.gov/pmc/artic les/PMC4097040/pdf/nihms522470.pdf> * page 3 - paragraph 3 * | 1-15 | TECHNICAL FIELDS SEARCHED (IPC) |

The present search report has been drawn up for all claims

| Place of search | Date of completion of the search | Examiner |
|---|---|---|
| Munich | 28 March 2022 | Young, Craig |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 21 20 5118

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2022

| Patent document cited in search report | | Publication date | Patent family member(s) | | | Publication date |
|---|---|---|---|---|---|---|
| WO 2015157650 | A1 | 15-10-2015 | CN | 106164270 | A | 23-11-2016 |
| | | | EP | 3129498 | A1 | 15-02-2017 |
| | | | ES | 2708205 | T3 | 09-04-2019 |
| | | | JP | 6562389 | B2 | 21-08-2019 |
| | | | JP | 2017510305 | A | 13-04-2017 |
| | | | KR | 20160138578 | A | 05-12-2016 |
| | | | US | 2017029810 | A1 | 02-02-2017 |
| | | | WO | 2015157650 | A1 | 15-10-2015 |
| WO 2015120447 | A1 | 13-08-2015 | EP | 3105348 | A1 | 21-12-2016 |
| | | | EP | 3653723 | A1 | 20-05-2020 |
| | | | US | 2015225713 | A1 | 13-08-2015 |
| | | | US | 2018371450 | A1 | 27-12-2018 |
| | | | WO | 2015120447 | A1 | 13-08-2015 |
| JP 2005224167 | A | 25-08-2005 | JP | 4690656 | B2 | 01-06-2011 |
| | | | JP | 2005224167 | A | 25-08-2005 |
| WO 2007036564 | A2 | 05-04-2007 | AT | 487791 | T | 15-11-2010 |
| | | | DE | 102005047736 | A1 | 05-04-2007 |
| | | | DK | 1934347 | T3 | 21-02-2011 |
| | | | EP | 1934347 | A2 | 25-06-2008 |
| | | | ES | 2356324 | T3 | 07-04-2011 |
| | | | PL | 1934347 | T3 | 31-05-2011 |
| | | | US | 2009011469 | A1 | 08-01-2009 |
| | | | WO | 2007036564 | A2 | 05-04-2007 |
| US 2006099605 | A1 | 11-05-2006 | NONE | | | |
| WO 2017203225 | A1 | 30-11-2017 | NONE | | | |
| US 2005026175 | A1 | 03-02-2005 | JP | 2005151975 | A | 16-06-2005 |
| | | | US | 2005026175 | A1 | 03-02-2005 |
| US 2005026159 | A1 | 03-02-2005 | EP | 1502951 | A1 | 02-02-2005 |
| | | | JP | 2005052142 | A | 03-03-2005 |
| | | | US | 2005026153 | A1 | 03-02-2005 |
| | | | US | 2005026159 | A1 | 03-02-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016147004 A

**Non-patent literature cited in the description**

- **CAVELIER L ; AMEUR A ; HAGGQVIST S ; HÖI-JER I ; CAHILL N ; OLSSON-STRÖMBERG U ; HERMANSON M et al.** Clonal distribution of BCR-ABL1 mutations and splice isoforms by single-molecule long-read RNA sequencing. *BMC Cancer,* 2015, vol. 15, 45